# EUROPÄISCHE PATENTSCHRIFT

(11) **EP 0 577 555 B1**
(45) Veröffentlichungstag und Bekanntmachung des Hinweises auf die Patenterteilung: **05.11.1997**
(21) Anmeldenummer: 93810443.7
(22) Anmeldetag: 22.06.1993
(51) Int. Cl.: C07C 69/65, A01N 37/06, A01N 43/40, A01N 43/82, C07C 69/96, C07D 213/647, C07C 205/34, C07C 321/28, C07D 285/08, C07C 255/54, C07C 233/20

(54) **Carbonsäurederivate**
Derivatives of carboxylic acids
Dérivés d'acides carboxyliques

(30) Priorität: 30.06.1992 CH 2038/92
(43) Veröffentlichungstag der Anmeldung: 05.01.1994
(73) Patentinhaber: Novartis AG, 4058 Basel (CH)
(72) Erfinder: Maienfisch, Peter, Dr., CH-4118 Rodersdorf (CH); Pitterna, Thomas, Dr., CH-4053 Basel (CH); Böger, Manfred, D-7858 Weil am Rhein 5 (DE)

(56) Entgegenhaltungen:
- EP-A- 0 432 861
- US-A- 4 950 666

## Beschreibung

Die vorliegende Erfindung betrifft neue Derivate von ω-Halogenvinylalkancarbonsäuren, Verfahren und Zwischenprodukte zu ihrer Herstellung, Schädlingsbekämpfungsmittel, welche diese Verbindungen enthalten, sowie ihre Verwendung bei der Kontrolle von Schädlingen.

Die erfindungsgemassen ω-Halogenvinylalkancarbonsäurederivate sind solche der Formel 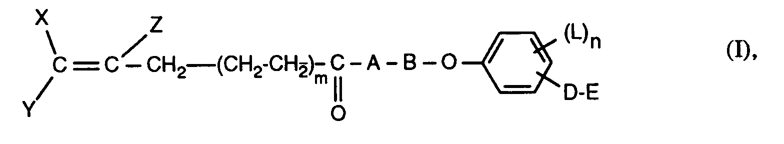 worin
A Sauerstoff, Schwefel oder -NR₁-;
B C₂-C₆-Alkylen,
   D-E -O-E, S-E, -O-CH₂-E, -O-C(=O)-E, -O-C(=O)-O-E, -O-C(=O)-N(H)-E oder -O-C(=S)-N(H)-E;
E Phenyl; durch ein bis drei Substituenten, ausgewählt aus der Gruppe, bestehend aus Halogen, C₁-C₄-Alkyl, Halogen-C₁-C₄-alkyl, C₁-C₄-Alkoxy, Halogen-C₁-C₄-alkoxy, Cyano, Nitro und Methylendioxy, substituiertes Phenyl; einen fünfgliedrigen aromatischen Heterocyclus mit ein bis drei Heteroatomen, ausgewählt aus der Gruppe, bestehend aus Stickstoff, Sauerstoff und Schwefel; einen durch ein oder zwei Substituenten, ausgewählt aus der Gruppe, bestehend aus Halogen, C₁-C₄-Alkyl und C₁-C₄-Halogenalkyl, substituierten fünfgliedrigen aromatischen Heterocyclus mit ein bis drei Heteroatomen, ausgewählt aus der Gruppe, bestehend aus Stickstoff, Sauerstoff und Schwefel; einen sechsgliedrigen aromatischen Heterocyclus mit ein bis drei Stickstoffatomen; oder einen durch ein oder zwei Substituenten, ausgewählt aus der Gruppe, bestehend aus Halogen, C₁-C₄-Alkyl und C₁-C₄-Halogenalkyl, substituierten sechsgliedrigen aromatischen Heterocyclus mit ein bis drei Stickstoffatomen;
L Halogen oder Methyl;
X Fluor;
Y Chlor oder Fluor;
Z Wasserstoff, Fluor oder Methyl;
m die Zahl null, eins, zwei, drei, vier oder fünf;
n die Zahl null, eins oder zwei und
R₁ Wasserstoff, C₁-C₄-Alkyl, Phenylthio oder Tolylthio bedeuten, in freier Form oder gegebenenfalls in Salzform.

In EP-A-0 432 861 werden Halogenolefinderivate als Wirkstoffe in Schädlingsbekämpfungsmitteln vorgeschlagen. Die biologischen Eigenschaften der in dieser Publikation beschriebenen Verbindungen vermögen auf dem Gebiet der Schädlingsbekämpfung jedoch nicht voll zu befriedigen, weshalb das Bedürfnis besteht, weitere Verbindungen mit schädlingsbekämpfenden Eigenschaften zur Verfügung zu stellen, wobei diese Aufgabe erfindungsgemäss durch die Bereitstellung der vorliegenden Verbindungen I gelöst wird.

Die erfindungsgemässen Verbindungen I schliessen gegebenenfalls, d. h. sofern mindestens ein basisches Zentrum in der betreffenden Verbindung I vorhanden ist, auch, insbesondere agrochemisch verträgliche, Säureadditionssalze ein. Beispiele geeigneter (anorganischer oder organischer) Säuren sind Salzsäure, Bromwasserstoffsäure, Schwefelsäure, Phosphorsäure, Salpetersäure, Säuren mit gleichem Zentralatom und höherer oder niedrigerer Oxidationsstufe, wie Perchlorsäure, salpetrige Säure oder phosphorige Säure, Essigsäure und Bernsteinsäure.

Die vor- und nachstehend verwendeten Allgemeinbegriffe haben, sofern nicht abweichend definiert, die nachfolgend aufgeführten Bedeutungen.

Bei den als Substituenten in Betracht kommenden Halogenatomen handelt es sich sowohl um Fluor und Chlor als auch um Brom und Iod, wobei Fluor und Chlor bevorzugt sind. Halogen ist dabei als selbständiger Substituent oder als Teil eines Substituenten zu verstehen, wie in Halogenalkyl oder Halogenalkoxy.

Kohlenstoffhaltige Gruppen und Verbindungen enthalten, sofern nicht abweichend definiert, jeweils vorzugsweise 1 bis und mit 4, insbesondere 1 oder 2, Kohlenstoffatome.

Die als Substituenten in Betracht kommenden Alkyl- und Alkoxyreste können geradkettig oder verzweigt sein. Als Beispiele solcher Alkylreste seien Methyl, Aethyl, Propyl, Isopropyl, Butyl, iso-Butyl, sek.-Butyl oder tert.-Butyl genannt. Als geeignete Alkoxyreste seien unter anderem genannt: Methoxy, Aethoxy, Propoxy, Isopropoxy oder Butoxy und ihre Isomeren.

Sind die als Substituenten in Betracht kommenden Alkyl-, Alkoxy- oder Phenylgruppen oder aromatischen Heterocyclen durch Halogen substituiert, so können sie nur teilweise oder auch perhalogeniert sein. Dabei gelten für Halogen, Alkyl und Alkoxy die oben gegebenen Definitionen. Beispiele der Alkylelemente dieser Gruppen sind das ein-bis dreifach durch Fluor, Chlor und/oder Brom substituierte Methyl, wie CHF₂ oder CF₃; das ein- bis fünffach durch Fluor, Chlor und/oder Brom substituierte Aethyl, wie CH₂CF₃, CF₂CF₃, CF₂CCl₃, CF₂CHCl₂, CF₂CHF₂, CF₂CFCl₂, CF₂CHBr₂, CF₂CHClF, CF₂CHBrF oder CClFCHClF; das ein- bis siebenfach durch Fluor, Chlor und/oder Brom substituierte Propyl oder Isopropyl, wie CH₂CHBrCH₂Br, CF₂CHFCF₃, CH₂CF₂CF₃ oder CH(CF₃)₂; und das ein- bis neunfach durch Fluor, Chlor und/oder Brom substituierte Butyl oder eines seiner Isomeren, wie CF(CF₃)CHFCF₃ oder CH₂(CF₂)₂CF₃. Die aromatischen Heterocyclen tragen die in Frage kommenden Substituenten vorzugsweise an einem der Kohlenstoffatome, welche zusammen mit den Heteroatomen das Ringgerüst bilden. Diese Ringe sind im allgemeinen ebenfalls über ein Kohlenstoffatom des Ringes an das Brückenglied D gebunden. Die fünfgliedrigen aromatischen Heterocyclen der erfindungsgemässen Definition des Restes E werden vorzugsweise durch die folgenden Grundstrukturen verkörpert: Pyrrol, Imidazol, Pyrazol, 1,2,3-Triazol, 1,2,4-Triazol, Oxazol, Isoxazol, Thiazol, Isothiazol, 1,2,3-Thiadiazol, 1,2,4-Thiadiazol, 1,3,4-Thiadiazol, 1,2,3-Oxadiazol, 1,2,4-Oxadiazol und 1,3,4-Oxadiazol. Als sechsgliedrige aromatische Heterocyclen kommen für E erfindungsgemäss Pyridin, Pyrimidin, Pyrazin, Pyridazin, 1,2,4-Triazin und 1,3,5-Triazin in Frage. Sind diese unter E definierten aromatischen Reste sowie Phenyl weiter substituiert, so können sie ein- bis dreifach durch den gleichen oder verschiedene der aufgezählten Substituenten substituiert sein. Vorzugsweise sind in den substituierten aromatischen Substituenten ein bis zwei Substituenten vorhanden. Insbesondere werden die aromatischen Reste der Definition -D-E auch durch die folgenden individualisierten Bedeutungen wiedergegeben:
Phenoxy,
Phenylthio,
3,5-Dichlorpyrid-2-yloxy,
Pyrid-2-yloxy,
Benzyloxy,
3-Chlorphenoxy,
3-Methyl-1,2,4-thiadiazol-5-yloxy,
4-Fluorphenoxy,
3-Fluorphenoxy,
2-Fluorphenoxy,
3-Chlor-5-trifluormethyl-pyrid-2-yloxy,
3-Chlor-5-(2,2-dichlor-1,1,2-trifluor-äthyl)-pyrid-2-yloxy,
3,5-Difluorphenoxy,
2-Aethoxymethyl-1,3,4-thiadiazol-5-ylmethoxy,
3-Isopropyl-1,2,4-thiadiazol-5-yloxy,
4-Chlorphenoxy,
2-Chlor-4-trifluormethyl-phenoxy,
5-Bromthien-2-ylmethoxy,
4-Trifluormethylphenoxy,
4-Aethylphenoxy,
4-Methoxyphenoxy,
4-Chlorphenylthio und
4-Chlorbenzyloxy,
4-Cyanobenzyloxy,
2-Nitrobenzyloxy,
3-Nitrobenzyloxy,
4-Nitrobenzyloxy,
4-Fluorbenzyloxy,
4-Trifluormethylbenzyloxy,
4-Methylbenzyloxy,
4-Methoxybenzyloxy
4-t-Butylbenzyloxy,
4-t-Butylphenoxy.

Die Reste -D-E können die ortho-, meta- oder para-Position des Phenylrestes besetzen. Bevorzugt ist die para-Stellung.

Durch die obligatorische ω-Stellung der Vinylgruppe in der zugrundeliegenden Alkenylcarbonsäure werden von der Definition der Formel I Derivate der But-3-ensäure, Hex-5-ensäure, Oct-7-ensäure, Dec-9-ensäure, Dodec-11-ensäure und Tetradec-13-ensäure umfasst.

Unter den Verbindungen der Formel I sind solche Untergruppen hervorzuheben, in denen entweder
a) A für Sauerstoff steht, oder
b) B für eine Aethylenbrücke steht, oder
c) A für eine Brücke -NH- steht, oder
d) n für die Zahl null steht, oder
e) D für -O- steht, oder
f) E für Phenyl; Pyridyl; Thiadiazolyl; durch ein oder zwei Substituenten, ausgewählt aus der Gruppe, bestehend aus Cyano, Nitro, Halogen, C₁-C₄-Alkyl und C₁-C₄-Halogenalkyl, substituiertes Phenyl; durch ein oder zwei Substituenten, ausgewählt aus der Gruppe, bestehend aus Halogen, C₁-C₄-Alkyl und Halogen-C₁-C₄-alkyl, substituiertes Pyridyl; oder C₁-C₄-Alkylthiazolyl steht, oder
g) m für die Zahl null, eins oder vier steht, oder
h) Y für Fluor und Z für Wasserstoff stehen, oder
i) D für -O-CH₂- steht, oder
k) D für -O-CO- oder -O-CO-O- steht.

Unter den Verbindungen der Untergruppe a) sind solche bevorzugt, worin n null, insbesondere, worin n null und m null, eins oder vier bedeutet, ganz besonders worin n null, m null, eins oder vier und B eine Aethylenbrücke bedeutet.

Weiterhin sind unter den Verbindungen der Untergruppe a) solche bevorzugt, worin n null ist und D Sauerstoff oder Schwefel bedeutet, insbesondere, worin n null ist, D Sauerstoff oder Schwefel bedeutet und B für eine Aethylenbrücke steht, ganz besonders bevorzugt, worin n null ist, D Sauerstoff oder Schwefel bedeutet, B für eine Aethylenbrücke steht und m null, eins oder vier bedeutet.

Ebenfalls sind unter den Verbindungen der Untergruppe a) solche bevorzugt, worin D -O-CH₂- bedeutet, insbesondere, worin D -O-CH₂- bedeutet, n null ist und B für eine Aethylenbrücke steht, ganz besonders bevorzugt, worin D -O-CH₂- bedeutet, n null ist, B für eine Aethylenbrücke steht und m null, eins oder vier bedeutet.

Ebenfalls sind unter den Verbindungen der Untergruppe a) solche bevorzugt, worin D die Gruppe -O-CO- oder -O-CO-O- bedeutet, besonders, worin D die Gruppe -O-CO- oder -O-CO-O- bedeutet und n null ist, besonders bevorzugt, worin D die Gruppe -O-CO- oder -O-CO-O- bedeutet, n null ist, B eine Aethylenbrücke darstellt und m null, eins oder vier bedeutet.

Unter den Verbindungen der Untergruppe e) sind solche bevorzugt, worin E für eine gegebenenfalls substituierte Phenyl-, Thiadiazolyl- oder Pyridyl-Gruppe steht, besonders worin E für Phenyl, Cyanophenyl, Nitrophenyl, Chlorphenyl, Fluorphenyl, Methylphenyl, t-Butylphenyl, Methoxyphenyl, Trifluormethylphenyl, Methylthiadiazolyl, Pyridyl, Chlorpyridyl, Trifluormethylpyridyl oder Chlor-trifluormethyl-pyridyl steht.

Unter den Verbindungen der Untergruppe f) sind solche bevorzugt, worin D - O-CH₂- bedeutet.

Unter den Verbindungen der Untergruppe f) sind weiterhin solche bevorzugt, worin D Sauerstoff bedeutet.

Unter den Verbindungen der Untergruppe f) sind auch solche bevorzugt, worin D Sauerstoff oder -O-CH₂- bedeutet und Y für Fluor und Z für Wasserstoff stehen, besonders worin D Sauerstoff oder -O-CH₂- bedeutet, Y für Fluor und Z für Wasserstoff stehen und n null ist, ganz besonders, worin D Sauerstoff oder - O-CH₂-bedeutet, Y für Fluor und Z für Wasserstoff stehen, n null ist und B eine Aethylenbrücke darstellt.

Ebenfalls sind unter den Verbindungen der Untergruppe f) solche bevorzugt, worin E für Phenyl, Cyanophenyl, Nitrophenyl, Chlorphenyl, Fluorphenyl, Methylphenyl, t-Butylphenyl, Methoxyphenyl, Trifluormethylphenyl, Methylthiadiazolyl, Pyridyl, Chlorpyridyl, Trifluormethylpyridyl oder Chlor-trifluormethyl-pyridyl steht, besonders worin D einerseits Sauerstoff, andererseits die Gruppe -O-CH₂-bedeutet.

Ganz besonders bevorzugt sind Verbindungen der Formel I, worin A für Sauerstoff, B für eine Aethylenbrücke, n für null, D für Sauerstoff, Schwefel oder -O-CH₂-, E für Phenyl; Pyridyl; Thiadiazolyl; durch ein oder zwei Substituenten, ausgewählt aus der Gruppe, bestehend aus Cyano, Nitro, Halogen, C₁-C₄-Alkyl und C₁-C₄-Halogenalkyl, substituiertes Phenyl; durch ein oder zwei Substituenten, ausgewählt aus der Gruppe, bestehend aus Halogen, C₁-C₄-Alkyl und Halogen-C₁-C₄-alkyl, substituiertes Pyridyl; oder C₁-C₄-Alkylthiazolyl, m für null, eins oder vier, Y für Fluor und Z für Wasserstoff steht.

Unter diesen bevorzugten Verbindungen der Formel I zeichnen sich durch ihre gute Wirksamkeit insbesondere diejenigen aus, worin entweder A Sauerstoff, B eine Aethylenbrücke, D Sauerstoff, Schwefel oder -O-CH₂-, m null, eins oder vier, n null, Y Fluor und Z Wasserstoff bedeuten; oder worin
A -NH-, B eine Aethylenbrucke, D Sauerstoff oder Schwefel, m null, eins oder vier, n null, Y Fluor und Z Wasserstoff bedeuten.

Als bevorzugte erfindungsgemässe Einzelverbindungen der Formel I sind zu nennen:
4,4-Difluorbut-3-ensäure-[2-(4-phenoxyphenoxy)äthylester],
4,4-Difluorbut-3-ensäure-[2-(4-benzyloxyphenoxy)äthylester],
4,4-Difluorbut-3-ensäure-[2-(4-{4-methyl}-benzyloxyphenoxy)äthylester],
6,6-Difluorhex-5-ensäure-[2-(4-phenoxyphenoxy)äthylester],
6,6-Difluorhex-5-ensäure-[2-(4-benzyloxyphenoxy)äthylester],
8,8-Difluoroct-7-ensäure-[2-(4-phenoxyphenoxy)äthylester],
8,8-Difluoroct-7-ensäure-[2-(4-benzyloxyphenoxy)äthylester],
10,10-Difluordec-9-ensäure-[2-(4-phenoxyphenoxy)äthylester],
10,10-Difluordec-9-ensäure-[2-(4-benzyloxyphenoxy)äthylester],
12,12-Difluordodec-11-ensäure-[2-(4-phenoxyphenoxy)äthylester],
12,12-Difluordodec-11-ensäure-[2-(4-benzyloxyphenoxy)äthylester].

Als weiterer Gegenstand der Erfindung ist ein Verfahren zur Herstellung der Verbindungen der Formel I, in freier Form oder gegebenenfalls in Salzform, beispielsweise dadurch gekennzeichnet, dass man
a) eine Verbindung der Formel 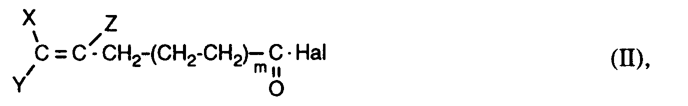 worin X, Y, Z und m die für die Formel I angegebenen Bedeutungen haben und Hal für Chlor oder Brom steht, gegebenenfalls in einem inerten Lösungsmittel und in Gegenwart eines säurebindenden Mittels, mit einer Verbindung der Formel 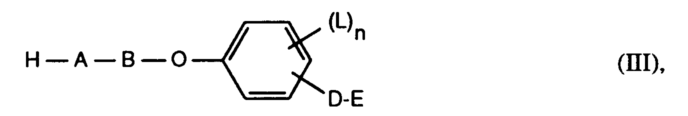 worin A, B, L, D, E und n die für die Formel I angegebenen Bedeutungen haben, oder gegebenenfalls einem Salz davon umsetzt oder
b) eine Verbindung der Formel 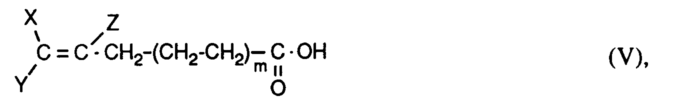 worin X, Y, Z und m die für die Formel I angegebenen Bedeutungen haben, gegebenenfalls in Gegenwart eines inerten Lösungsmittels und eines Katalysators oder eines wasserentziehenden Mittels, mit einer Verbindung der Formel III oder gegebenenfalls einem Salz davon umsetzt
und jeweils, wenn erwünscht, eine verfahrensgemäss erhältliche freie Verbindung der Formel I in ein Salz oder ein verfahrensgemäss erhältliches Salz einer Verbindung der Formel I in die freie Verbindung der Formel I oder in ein anderes Salz überführt.

Die Umsetzung des Verfahrens a) erfolgt vorzugsweise in einem inerten, hydroxylgruppenfreien Lösungsmittel in Anwesenheit einer organischen Base, wie zum Beispiel Pyridin, 4-Dimethylaminopyridin, Lutidin, Collidin, Trialkylamin, N,N-Dialkylanilin, oder einer bicyclischen, nicht nucleophilen Base wie 1,4-Diazabicyclo[2.2.2]octan (DABCO), 1,5-Diazabicyclo[4.3.0]non-5-en (DBN) oder 1,8-Diazabicyclo[5.4.0]undec-7-en (1,5-5) (DBU). Die Reaktion wird im allgemeinen bei Temperaturen von -30°C bis +70°C, vorzugsweise von -10°C bis +50°C durchgeführt Man arbeitet dabei zweckmässigerweise in Gegenwart eines reaktionsinerten Lösungsmittels oder Lösungsmittelgemisches. Es eignen sich hierfür beispielsweise aliphatische und aromatische Kohlenwasserstoffe wie Benzol, Toluol, Xylole, Petroläther, Hexan; halogenierte Kohlenwasserstoffe wie Chlorbenzol, Methylenchlorid, Aethylenchlorid, Chloroform, Tetrachlorkohlenwasserstoff, Tetrachloräthylen; Aether und ätherartige Verbindungen wie Dialkyläther (Diäthyläther, Diisopropyläther, tert-Butylmethyläther usw.), Anisol, Dioxan, Tetrahydrofuran; Nitrile wie Acetonitril, Propionitril; Ester wie Aethylacetat (Essigsäureäthylester), Propylacetat oder Butylacetat; Ketone wie Aceton, Diäthylketon, Methyläthylketon; und Gemische solcher Lösungsmittel untereinander. Man kann die Reaktion aber auch im Ueberschuss einer der oben genannten Basen durchführen, oder im Falle, dass die Verbindung der Formel III ein Amin darstellt (A = NR₁), kann anstelle der Base auch ein zweites Aequivalent oder auch ein grösserer Ueberschuss der Verbindung der Formel III eingesetzt werden. Die Umsetzung wird unter dem Druck der Umgebung durchgeführt, wenngleich sie auch bei erhöhtem oder vermindertem Druck durchgeführt werden könnte.

Bei der Verfahrensvariante b) wird die Reaktion vorteilhafterweise in Gegenwart von für Veresterungen üblichen, wasserabspaltenden Reagenzien durchgeführt, wie zum Beispiel in Anwesenheit eines Carbodiimids [Dicyclohexylcarbodiimid (DCC)] oder eines 1-Alkyl-2-halogen-pyridiniumsalzes wie 1-Methyl-2-chlorpyridiniumjodid. Zweckmässigerweise wird die Reaktion dann in Gegenwart eines reaktionsinerten Lösungsmittels oder Lösungsmittelgemisches bei Temperaturen von -30°C bis +70°C, vorzugsweise -10°C bis +50°C durchgeführt. Man arbeitet bevorzugt in Gegenwart einer Base wie beispielsweise in Gegenwart eines organischen Amins wie eines Trialkylamins (Trimethylamin, Triäthylamin, Tripropylamin oder Diisopropyläthylamin), eines Pyridins (Pyridin selbst, 4-Dimethylaminopyridin oder 4-Pyrrolidinopyridin), eines Morpholins (N-Methylmorpholin) oder eines N,N-Dialkylanilins (N,N-Dimethylanilin oder N-Methyl-N-äthylanilin). Als Lösungsmittel eignen sich hierfür beispielsweise aliphatische und aromatische Kohlenwasserstoffe wie Benzol, Toluol, Xylole, Petroläther, Hexan; halogenierte Kohlenwasserstoffe wie Chlorbenzol, Methylenchlorid, Aethylenchlorid, Chloroform, Tetrachlorkohlenstoff, Tetrachloräthylen; Aether und ätherartige Verbindungen wie Dialkyläther (Diäthyläther, Diisopropyläther, tert.-Butylmethyläther usw.), Anisol, Dioxan, Tetrahydrofuran; Nitrile wie Acetonitril, Propionitril; Ester wie Aethylacetat (Essigsäureäthylester), Propylacetat oder Butylacetat; und Gemische solcher Lösungsmittel untereinander.

Stellt die Verbindung der Formel III einen Alkohol dar (A = O) so kann die VerfahrensVariante b) auch in Gegenwart eines Säurekatalysators, wie zum Beispiel H₂SO₄, HCl oder einer Sulfonsäure wie Methansulfonsäure oder p-Toluolsulfonsäure, durchgeführt werden. Man arbeitet dabei vorteilhafterweise mit einem Ueberschuss des Alkohols der Formel III. Bei diesem Verfahren kann freiwerdendes Wasser kontinuierlich aus dem Reaktionsgemisch entfernt werden. Eine übliche Methode dazu ist das Entfernen des Reaktionsproduktes Wasser durch Abdestillieren eines Azeotrops des Lösungsmittels mit Wasser. Dazu geeignete Lösungsmittel sind Benzol, Toluol, Xylol, Methylenchlorid oder Chloroform.

Die Umwandlung von freien Verbindungen I in Salze und von Salzen in freie Verbindungen I oder in andere Salze erfolgt in üblicher Weise, z. B. durch Behandlung einer freien Verbindung I mit einer Säure oder eines Salzes mit einer Base.

Grundsätzlich sind die verschiedenen Derivate der Formel I auch durch Umesterung oder Amidierung aus den leicht zugänglichen Niederalkylestern der ω-Halogenvinylalkancarbonsäure der Formel V erhältlich.

Beispielsweise können die Derivate des Estertyps der Formel I (A = O oder S) durch basen- oder säurekatalysierte Umesterung der Niederalkylester der Formel 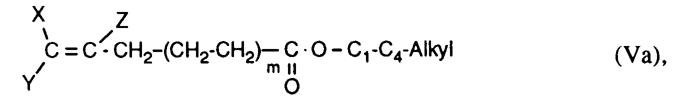 worin X, Y, Z und m die für die Formel I angegebenen Bedeutungen haben, mit den Alkoholen oder Mercaptanen der Formel 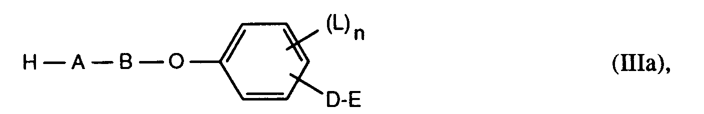 worin B, D, E, L und n die für die Formel I angegebenen Bedeutungen haben und A für Sauerstoff oder Schwefel steht, erhalten werden. Als Säurekatalysatoren besonders geeignet sind HCl, H₂SO₄ oder eine Sulfonsäure. Bei der basenkatalysierten Umesterung verwendet man vorzugsweise als Base das Natrium- oder Kaliumalkoholat des Alkohols oder Mercaptans der Formel IIIa, welches aus IIIa beispielsweise durch Zugabe von Natrium- oder Kaliumhydrid zugänglich ist. Die Umesterungsreaktion wird vorzugsweise bei Temperaturen zwischen -20°C und +120°C, insbesondere zwischen 0°C und +100°C durchgeführt. Die Alkohol- oder Mercaptankomponente IIIa wird mit Vorteil im Ueberschuss verwendet. Als Lösungsmittel kommen Aether, wie Diäthyläther, Diisopropyläther, Dioxan oder Tetrahydrofuran, halogenierte Kohlenwasserstoffe oder aliphatische oder aromatische Kohlenwasserstoffe in Frage.

Derivate des Amidtyps der Formel I (A = NR₁) werden aus den Niederalkylestern der Formel Va erhalten, indem man diese mit einem Amin der Formel 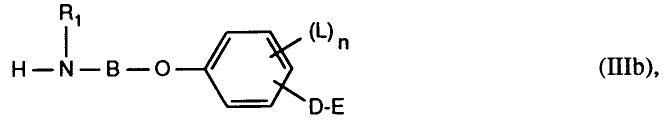 worin R₁, B, D, E, L und n die für die Formel I angegebenen Bedeutungen haben, oder einem Salz davon umsetzt. Die Amidierungsreaktionen führt man bei Temperaturen zwischen 0°C und +120°C durch. Mit Vorteil werden die Reaktanden in einem inerten Lösungsmittel oder Lösungsmittelgemisch umgesetzt. Es eignen sich hierfür beispielsweise aliphatische und aromatische Kohlenwasserstoffe wie Benzol, Toluol, Xylole, Petroläther, Hexan; halogenierte Kohlenwasserstoffe wie Chlorbenzol, Methylenchlorid, Aethylenchlorid, Chloroform, Tetrachlorkohlenstoff, Tetrachloräthylen; Aether und ätherartige Verbindungen wie Dialkyläther (Diäthyläther, Diisopropyläther, tert.-Butylmethyläther usw.), Anisol, Dioxan, Tetrahydrofuran; Nitrile wie Acetonitril, Propionitril; Alkohole wie Methanol, Aethanol, Propanol, Isopropanol; oder Wasser. Die Aminkomponente IIIb wird mit Vorteil im Ueberschuss verwendet.

Die Verbindungen der Formeln II, III, IIIa, IIIb, V und Va und ihre Herstellung sind aus der Literatur bekannt. Die Verbindungen II und V können nach den in US-4,950,666 und EP-A-0 432 861 beschriebenen Verfahren hergestellt werden. Die Reaktionsbedingungen zur Synthese der freien Säure aus einem Ester und die nachfolgende fakultative Ueberführung dieser Säure V in das Säurehalogenid der Formel II entsprechen den üblichen Bedingungen für eine säure- oder base-katalysierte Hydrolyse, beziehungsweise für eine Halogenierung einer Carbonsäure. Die noch nicht in der Literatur beschriebenen Verbindungen der Formeln III, IIIa und IIIb können analog zu den bekannten Verfahren nach üblichen Synthesemethoden erhalten werden.

In einem weiteren Verfahren können diejenigen Verbindungen der Formel I, worin Z für Wasserstoff oder Methyl steht, in freier Form oder in Salzform, erhalten werden, indem man ein ω-Carbonylalkancarbonsäurederivat der Formel 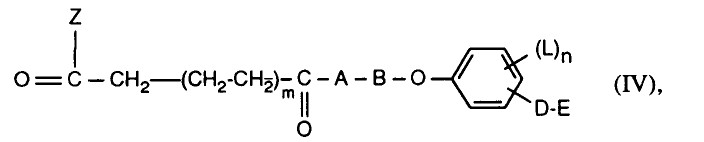 worin A, B, L, D, E, m und n die für die Formel I angegebenen Bedeutungen haben und Z für Wasserstoff oder Methyl steht, oder gegebenenfalls ein Salz davon in einem inerten Lösungsmittel in Gegenwart eines trisubstituierten Phosphans, wie P(C₆H₅)₃, P(C₂H₅)₃, P[N(C₂H₅)₂]₃ oder P[N(CH₃)₂]₃, mit einem Halogenmethan, wie CF₂Br₂, CCl₂F₂ oder CCl₃F, oder einem Trihalogenessigsäure-Alkalisalz, wie ClF₂C-CO-ONa oder Cl₂FC-CO-ONa, umsetzt.

Für die Durchfürhung dieser Reaktionsvariante eignen sich als Lösungsmittel bevorzugt Aether, wie Diglyme, Triglyme oder Tetraglyme, oder Dimethylacetamid. Dieser Reaktionsschritt wird im allgemeinen bei einer Temperatur zwischen 0°C und +150°C, vorzugsweise zwischen +20°C und +100°C durchgeführt.

Die Verbindungen der Formel IV und gegebenenfalls ihre Salze sind neu. Sie wurden speziell für die Synthese der Wirkstoffe der Formel I entwickelt. Die Verbindungen der Formel IV bilden daher einen Bestandteil der vorliegenden Erfindung.

Die Verbindungen der Formel IV können nach an sich bekannten Verfahren aus in der Literatur beschriebenen oder käuflichen Produkten hergestellt werden. Beispielsweise kann eine Verbindung der Formel IV hergestellt werden, indem man eine Säure oder ein Säurehalogenid der Formel 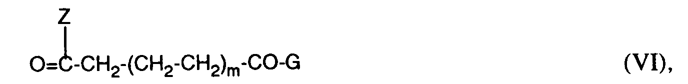 worin m die für die Formel I angegebene Bedeutung hat, G Hydroxy oder Halogen, vorzugsweise Chlor oder Brom, und Z Wasserstoff oder Methyl bedeuten, gegebenenfalls in Gegenwart eines inerten Lösungsmittels und eines Katalysators oder einen wasserentziehenden Mittels mit einer Verbindung der Formel III oder gegebenenfalls einem Salz davon umsetzt. Die Reaktionsbedingungen dieser Veresterung oder Amidierung entsprechen denen der Verfahrensvarianten a) und b).

Die Verbindungen de Formel VI sind bekannt oder können analog zu bekannten Verfahren hergestellt werden.

In einem weiteren Verfahren können die Verbindungen der engeren Untergruppe der Formel 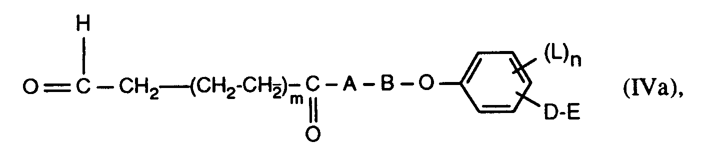 worin A, B, D, E, L, m und n die für die Formel I angegebenen Bedeutungen haben, und gegebenenfalls ihre Salze erhalten werden, indem man ein ω-Halogenalkancarbonsäurederivat der Formel 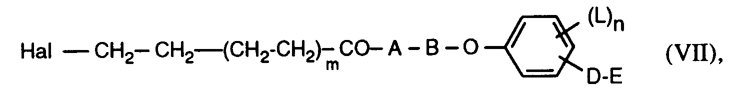 worin A, B, D, E, L, m und n die für die Formel I angegebenen Bedeutungen haben und Hal Chlor, Brom oder Iod bedeutet, oder gegebenenfalls ein Salz davon in Dimethylsulfoxid erhitzt. Vorzugsweise betragen die Temperaturen bei dieser Reaktion zwischen +50°C und +180°C.

Die Verbindungen der Formel VII können beispielsweise durch Veresterung oder Amidierung analog zu den Verfahrensvarianten a) und b) aus einer Verbindung der Formel

Hal-CH₂-CH₂-(CH₂-CH₂)ₘ-CO-G (VIII),

worin m die für die Formel I angegebene Bedeutung hat, G Hydroxy oder Halogen, vorzugsweise Chlor oder Brom, und Hal Chlor, Brom oder Iod bedeuten, erhalten werden.

Die Verbindungen der Formel VIII sind bekannt oder lassen sich analog bekannten Verfahren herstellen.

Verbindungen der Formel I, worin Y für Chlor steht, können als Doppelbindungsisomere in der E- oder Z-Form vorliegen. Die reinen Doppelbindungsisomeren können mit Hilfe physikalischer Methoden, wie Destillation oder Fest-Flüssig-Chromatographie, aus dem Isomerengemisch abgetrennt werden.

Die erfindungsgemässen Verbindungen I sind bei günstiger Warmblüter-, Fisch- und Pflanzenverträglichkeit wertvolle Wirkstoffe auf dem Gebiet der Schädlingsbekämpfung. Insbesondere wirken die erfindungsgemässen Wirkstoffe gegen Insekten und Spinnentiere, wie sie an Nutz- und Zierpflanzen in der Landwirtschaft und im Gartenbau, insbesondere in Reis-, Baumwoll-, Gemüse- und Obstpflanzungen, und im Forst vorkommen. Die Verbindungen I eignen sich besonders zur Bekämpfung von Insekten in Reis-, Obst- und Gemüsekulturen, insbesondere von pflanzenschädigenden Insekten, wie Aphis craccivora, Nilaparvata lugens und Nephotettix cincticeps. Weitere Anwendungsgebiete der erfindungsgemässen Wirkstoffe sind der Vorrats- und Materialschutz sowie im Hygienesektor insbesondere der Schutz von Haus- und Nutztieren. Die Verbindungen I sind gegen alle oder einzelne Entwicklungsstadien von normal sensiblen, aber auch von resistenten Arten von Schädlingen wirksam. Dabei kann sich ihre Wirkung z. B. in einer Abtötung der Schädlinge, welche unmittelbar oder erst nach einiger Zeit, beispielsweise bei einer Häutung, eintritt, oder in einer verminderten Eiablage und/oder Schlupfrate zeigen.

Zu den oben erwähnten Schädlingen gehören:
aus der Ordnung Lepidoptera zum Beispiel
Acleris spp., Adoxophyes spp., Aegeria spp., Agrotis spp., Alabama argillaceae, Amylois spp., Anticarsia gemmatalis, Archips spp., Argyrotaenia spp., Autographa spp., Busseola fusca, Cadra cautella, Carposina nipponensis, Chilo spp., Choristoneura spp., Clysia ambiguella, Cnaphalocrocis spp., Cnephasia spp., Cochylis spp., Coleophora spp., Crocidolomia binotalis, Cryptophlebia leucotreta, Cydia spp., Diatraea spp., Diparopsis castanea, Earias spp., Ephestia spp., Eucosma spp., Eupoecilia ambiguella, Euproctis spp., Euxoa spp., Grapholita spp., Hedya nubiferana, Heliothis spp., Hellula undalis, Hyphantria cunea, Keiferia lycopersicella, Leucoptera scitella, Lithocollethis spp., Lobesia botrana, Lymantria spp., Lyonetia spp., Malacosoma spp., Mamestra brassicae, Manduca sexta, Operophtera spp., Ostrinia nubilalis, Pammene spp., Pandemis spp., Panolis flammea, Pectinophora gossypiella, Phthorimaea operculella, Pieris rapae, Pieris spp., Plutella xylostella, Prays spp., Scirpophaga spp., Sesamia spp., Sparganothis spp., Spodoptera spp., Synanthedon spp., Thaumetopoea spp., Tortrix spp., Trichoplusia ni und Yponomeuta spp.;
aus der Ordnung Coleoptera zum Beispiel
Agriotes spp., Anthonomus spp., Atomaria linearis, Chaetocnema tibialis, Cosmopolites spp., Curculio spp., Dermestes spp., Diabrotica spp., Epilachna spp., Eremnus spp., Leptinotarsa decemlineata, Lissorhoptrus spp., Melolontha spp., Orycaephilus spp., Otiorhynchus spp., Phlyctinus spp., Popillia spp., Psylliodes spp., Rhizopertha spp., Scarabeidae, Sitophilus spp., Sitotroga spp., Tenebrio spp., Tribolium spp. und Trogoderma spp.;
aus der Ordnung Orthoptera zum Beispiel
Blatta spp., Blattella spp., Gryllotalpa spp., Leucophaea maderae, Locusta spp., Periplaneta spp. und Schistocerca spp.;
aus der Ordnung Isoptera zum Beispiel
Reticulitermes spp.;
aus der Ordnung Psocoptera zum Beispiel
Liposcelis spp.;
aus der Ordnung Anoplura zum Beispiel
Haematopinus spp., Linognathus spp., Pediculus spp., Pemphigus spp. und Phylloxera spp.;
aus der Ordnung Mallophaga zum Beispiel
Damalinea spp. und Trichodectes spp.;
aus der Ordnung Thysanoptera zum Beispiel
Frankliniella spp., Hercinothrips spp., Taeniothrips spp., Thrips palmi, Thrips tabaci und Scirtothrips aurantii;
aus der Ordnung Heteroptera zum Beispiel
Cimex spp., Distantiella theobroma, Dysdercus spp., Euchistus spp., Eurygaster spp., Leptocorisa spp., Nezara spp., Piesma spp., Rhodnius spp., Sahlbergella singularis, Scotinophara spp. und Triatoma spp.;
aus der Ordnung Homoptera zum Beispiel
Aleurothrixus floccosus, Aleyrodes brassicae, Aonidiella spp., Aphididae, Aphis spp., Aspidiotus spp., Bemisia tabaci, Ceroplaster spp., Chrysomphalus aonidium, Chrysomphalus dictyospermi, Coccus hesperidum, Empoasca spp., Eriosoma larigerum, Erythroneura spp., Gascardia spp., Laodelphax spp., Lecanium corni, Lepidosaphes spp., Macrosiphus spp., Myzus spp., Nephotettix spp., Nilaparvata spp., Paratoria spp., Pemphigus spp., Planococcus spp., Pseudaulacaspis spp., Pseudococcus spp., Psylla spp., Pulvinaria aethiopica, Quadraspidiotus spp., Rhopalosiphum spp., Saissetia spp., Scaphoideus spp., Schizaphis spp., Sitobion spp., Trialeurodes vaporariorum, Trioza erytreae und Unaspis citri;
aus der Ordnung Hymenoptera zum Beispiel
Acromyrmex, Atta spp., Cephus spp., Diprion spp., Diprionidae, Gilpinia polytoma, Hoplocampa spp., Lasius spp., Monomorium pharaonis, Neodiprion spp., Solenopsis spp. und Vespa spp.;
aus der Ordnung Diptera zum Beispiel
Aedes spp., Antherigona soccata, Bibio hortulanus, Calliphora erythrocephala, Ceratitis spp., Chrysomyia spp., Culex spp., Cuterebra spp., Dacus spp., Drosophila melanogaster, Fannia spp., Gastrophilus spp., Glossina spp., Hypoderma spp., Hyppobosca spp., Liriomyza spp., Lucilia spp., Melanagromyza spp., Musca spp., Oestrus spp., Orseolia spp., Oscinella frit, Pegomyia hyoscyami, Phorbia spp., Rhagoletis pomonella, Sciara spp., Stomoxys spp., Tabanus spp., Tannia spp. und Tipula spp.;
aus der Ordnung Siphonaptera zum Beispiel
Ceratophyllus spp. und Xenopsylla cheopis;
aus der Ordnung Thysanura zum Beispiel
Lepisma saccharina und
aus der Ordnung Acarina zum Beispiel
Acarus siro, Aceria sheldoni, Aculus schlechtendali, Amblyomma spp., Argas spp., Boophilus spp., Brevipalpus spp., Bryobia praetiosa, Calipitrimerus spp., Chorioptes spp., Dermanyssus gallinae, Eotetranychus carpini, Eriophyes spp., Hyalomma spp., Ixodes spp., Olygonychus pratensis, Ornithodoros spp., Panonychus spp., Phyllocoptruta oleivora, Polyphagotarsonemus latus, Psoroptes spp., Rhipicephalus spp., Rhizoglyphus spp., Sarcoptes spp., Tarsonemus spp. und Tetranychus spp..

Insbesondere eignen sich die Verbindungen der Formel I zur Kontrolle der Schädlinge in Baumwolle-, Gemüse-, Obst- und Reiskulturen, wie Spinnmilben, Blattläusen, Falterraupen und Reiszikaden. Hauptsächlich können dabei Spinnmilben wie Panonychus ulmi, Blattläuse wie Aphis craccivora, Falterraupen wie die von Heliothis virescens und Reiszikaden wie Nilaparvata lugens oder Nephotettix cincticeps kontrolliert werden.

Die gute pestizide Wirkung der erfindungsgemässen Verbindungen I entspricht einer Abtötungsrate (Mortalität) von mindestens 50-60 % der erwähnten Schädlinge.

Die Wirkung der erfindungsgemässen Verbindungen I und der sie enthaltenden Mittel lässt sich durch Zusatz von anderen Insektiziden und/oder Akariziden wesentlich verbreitern und an gegebene Umstände anpassen. Als Zusätze kommen zum Beispiel Vertreter der folgenden Wirkstoffklassen in Betracht: Organische Phosphorverbindungen, Nitrophenole und Derivate, Formamidine, Harnstoffe, Carbamate, Pyrethroide, chlorierte Kohlenwasserstoffe und Bacillus thuringiensis-Präparate.

Die Verbindungen der Formel I werden in unveränderter Form oder vorzugsweise zusammen mit den in der Formulierungstechnik üblichen Hilfsmitteln eingesetzt und können daher beispielsweise zu emulgierbaren Konzentraten, direkt versprüh- oder verdünnbaren Lösungen, verdünnten Emulsionen, Spritzpulvern, löslichen Pulvern, Stäubemitteln, Granulaten, auch Verkapselungen in polymeren Stoffen in bekannter Weise verarbeitet werden. Die Anwendungsverfahren, wie Versprühen, Vernebeln, Verstäuben, Verstreuen oder Giessen, werden ebenso wie die Mittel den angestrebten Zielen und den gegebenen Verhältnissen entsprechend gewählt.

Die Formulierung, das heisst die den Wirkstoff der Formel I, beziehungsweise Kombinationen dieser Wirkstoffe mit anderen Insektiziden oder Akariziden, und gegebenenfalls einen festen oder flüssigen Zusatzstoff enthaltenden Mittel, Zubereitungen oder Zusammensetzungen, werden in bekannter Weise hergestellt, zum Beispiel durch inniges Vermischen und/oder Vermahlen der Wirkstoffe mit Streckmitteln, wie beispielsweise mit Lösungsmitteln, festen Trägerstoffen, und gegebenenfalls oberflächenaktiven Verbindungen (Tensiden).

Als Lösungsmittel können in Frage kommen: Aromatische Kohlenwasserstoffe, bevorzugt die Fraktionen C₈ bis C₁₂ von Alkylbenzolen wie Xylolgemische oder alkylierte Naphthaline, aliphatische oder cycloaliphatische Kohlenwasserstoffe, wie Cyclohexan, Paraffine oder Tetrahydronaphthalin, Alkohole wie Aethanol, Propanol oder Butanol, und Glykole sowie deren Aether und Ester, wie Propylenglykol, Dipropylenglykoläther, Aethylenglykol, Aethylenglykolmonomethyl- oder -äthyläther, Ketone, wie Cyclohexanon, Isophoron oder Diacetanolalkohol, starke polare Lösungsmittel, wie N-Methyl-2-pyrrolidon, Dimethylsulfoxid oder Dimethylformamid oder Wasser, Pflanzenöle, wie Raps-, Rizinus-, Kokosnuss- oder Sojaöl; gegebenenfalls auch Silikonöle.

Als feste Trägerstoffe, beispielsweise für Stäubemittel und dispergierbare Pulver, werden in der Regel natürliche Gesteinsmehle verwendet, wie Calcit, Talkum, Kaolin, Montmorillonit oder Attapulgit. Zur Verbesserung der physikalischen Eigenschaften können auch hochdisperse Kieselsäuren oder hochdisperse saugfähige Polymerisate zugesetzt werden. Als gekörnte, adsorptive Granulatträger kommen poröse Typen, wie Bimsstein, Ziegelbruch, Sepiolit oder Bentonit, als nicht sorptive Trägermaterialien Calcit oder Sand in Frage. Darüberhinaus kann eine Vielzahl von granulierten Materialien anorganischer oder organischer Natur, wie insbesondere Dolomit oder zerkleinerte Pflanzenrückstände, verwendet werden.

Als oberflächenaktive Verbindungen kommen je nach Art des zu formulierenden Wirkstoffes der Formel I oder der Kombinationen dieser Wirkstoffe mit andern Insektiziden oder Akariziden nichtionogene, kation- und/oder anionaktive Tenside mit guten Emulgier-, Dispergier- und Netzeigenschaften in Betracht. Unter Tensiden sind auch Tensidgemische zu verstehen.

Geeignete anionische Tenside können sowohl sogenannte wasserlösliche Seifen als auch wasserlösliche synthetische oberflächenaktive Verbindungen sein.

Als Seifen eignen sich die Alkali-, Erdalkali- oder gegebenenfalls substituierten Ammoniumsalze von höheren Fettsäuren (C₁₀-C₂₂), wie die Natrium- oder Kalium-Salze der Oel- oder Stearinsäure, oder von natürlichen Fettsäuregemischen, die beispielsweise aus Kokosnuss- oder Tallöl gewonnen werden können. Ferner sind als Tenside auch die Fettsäuremethyl-taurin-salze zu erwähnen.

Häufiger werden jedoch sogenannte synthetische Tenside verwendet, insbesondere Fettsulfonate, Fettsulfate, sulfonierte Benzimidazolderivate oder Alkylarylsulfonate.

Die Fettsulfonate oder -sulfate liegen in der Regel als Alkali-, Erdalkali-oder gegebenenfalls substituierte Ammoniumsalze vor und weisen im allgemeinen einen Alkylrest mit 8 bis 22 C-Atomen auf, wobei Alkyl auch den Alkylteil von Acylresten einschliesst, beispielsweise das Natrium- oder Calcium-Salz der Ligninsulfonsäure, des Dodecylschwefelsäureesters oder eines aus natürlichen Fettsäuren hergestellten Fettalkoholsulfatgemisches. Hierher gehören auch die Salze der Schwefelsäureester und Sulfonsäuren von Fettalkohol-Aethylenoxid-Addukten. Die sulfonierten Benzimidazolderivate enthalten vorzugsweise 2 Sulfonsäuregruppen und einen Fettsäurerest mit etwa 8-22 C-Atomen. Alkylarylsulfonate sind zum Beispiel die Natrium-, Calcium- oder Triäthanolaminsalze der Dodecylbenzolsulfonsäure, der Dibutylnaphthalinsulfonsäure oder eines Naphthalinsulfonsäure-Formaldehydkondensationsproduktes. Ferner kommen auch entsprechende Phosphate, wie zum Beispiel Salze des Phosphorsäureesters eines p-Nonylphenol-(4-14)-Aethylenoxid-Adduktes oder Phospholipide in Frage.

Als nichtionische Tenside kommen in erster Linie Polyglykolätherderivate von aliphatischen oder cycloaliphatischen Alkoholen, gesättigten oder ungesättigten Fettsäuren und Alkylphenolen in Frage, die 3 bis 30 Glykoläthergruppen und 8 bis 20 Kohlenstoffatome im (aliphatischen) Kohlenwasserstoffrest und 6 bis 18 Kohlenstoffatome im Alkylrest der Alkylphenole enthalten können. Weiterhin geeignete nichtionische Tenside sind die wasserlöslichen 20 bis 250 Aethylenglykoläthergruppen und 10 bis 100 Propylenglykoläthergruppen enthaltenden Polyäthylenoxid-Addukte an Polypropylenglykol, Aethylendiaminopolypropylenglykol und Alkylpolypropylenglykol mit 1 bis 10 Kohlenstoffatomen in der Alkylkette. Die genannten Verbindungen enthalten üblicherweise pro Propylenglykol-Einheit 1 bis 5 Aethylenglykol-Einheiten.

Als Beispiele nichtionischer Tenside seien Nonylphenolpolyäthoxyäthanole, Ricinusölpolyglykoläther, Polypropylen-Polyäthylenoxid-Addukte, Tributylphenoxypolyäthoxyäthanol, Polyäthylenglykol und Octylphenoxypolyäthoxyäthanol erwähnt. Ferner kommen auch Fettsäureester von Polyoxyäthylensorbitan, wie das Polyoxyäthylensorbitan-trioleat in Betracht.

Bei den kationischen Tensiden handelt es sich vor allem um quarternäre Ammoniumsalze, welche als N-Substituenten mindestens einen Alkylrest mit 8 bis 22 C-Atomen enthalten und als weitere Substituenten niedrige, gegebenenfalls halogenierte Alkyl-, Benzyl- oder niedrige Hydroxyalkylreste aufweisen. Die Salze liegen vorzugsweise als Halogenide, Methylsulfate oder Aethylsulfate vor, beispielsweise das Stearyltrimethylammoniumchlorid oder das Benzyl-di-(2-chloräthyl)-äthylammoniumbromid.

Die vorstehend aufgeführten Tenside sind nur als Beispiele anzusehen; in der einschlägigen Literatur werden viele weitere in der Formulierungstechnik gebräuchliche und erfindungsgemäss geeignete Tenside beschrieben.

Die pestiziden Zubereitungen enthalten in der Regel 0,1 bis 99%, insbesondere 0,1 bis 95%, an Wirkstoff I oder an der Kombination dieses Wirkstoffs mit anderen Insektiziden und/oder Akariziden und 1 bis 99,9%, insbesondere 5 bis 99,9%, eines festen oder flüssigen Hilfsstoffes, wobei in der Regel 0 bis 25%, insbesondere 0,1 bis 20%, der Zubereitungen Tenside sein können (% bedeutet jeweils Gewichtsprozent). Während als Handelsware eher konzentrierte Mittel bevorzugt werden, verwendet der Endverbraucher in der Regel verdünnte Zubereitungen, die wesentlich geringere Wirkstoffkonzentrationen aufweisen. Typische Anwendungskonzentrationen liegen zwischen 0,1 und 1000 ppm, vorzugsweise zwischen 0,1 und 500 ppm. Die Aufwandmengen pro Hektar betragen im allgemeinen 1 bis 1000 g Wirkstoff pro Hektar, vorzugsweise 25 bis 500 g/ha.

Insbesondere setzen sich bevorzugte Formulierungen folgendermassen zusammen (% = Gewichtsprozent):

| Emulgierbare Konzentrate: | |
|---|---|
| Wirkstoff: | 1 bis 90 %, bevorzugt 5 bis 20 % |
| Tensid: | 1 bis 30 %, vorzugsweise 10 bis 20 % |
| flüssiger Trägerstoff: | 5 bis 94 %, vorzugsweise 70 bis 85 % |

| Stäube: | |
|---|---|
| Wirkstoff: | 0,1 bis 10 %, vorzugsweise 0,1 bis 1 % |
| fester Trägerstoff: | 99,9 bis 90 %, vorzugsweise 99,9 bis 99 % |

| Suspensions-Konzentrate: | |
|---|---|
| Wirkstoff: | 5 bis 75 %, vorzugsweise 10 bis 50 % |
| Wasser: | 94 bis 24 %, vorzugsweise 88 bis 30 % |
| Tensid: | 1 bis 40 %, vorzugsweise 2 bis 30 % |

| Benetzbare Pulver: | |
|---|---|
| Wirkstoff: | 0,5 bis 90 %, vorzugsweise 1 bis 80 % |
| Tensid: | 0,5 bis 20 %, vorzugsweise 1 bis 15 % |
| fester Trägerstoff: | 5 bis 95 %, vorzugsweise 15 bis 90 % |

| Granulate: | |
|---|---|
| Wirkstoff: | 0,5 bis 30 %, vorzugsweise 3 bis 15 % |
| fester Trägerstoff: | 99,5 bis 70 %, vorzugsweise 97 bis 85 % |

Die Zubereitungen können auch weitere Hilfsstoffe, wie Stabilisatoren, z. B. gegebenenfalls epoxidierte Pflanzenöle (z. B. epoxidiertes Kokosnussöl, Rapsöl oder Sojaöl), Entschäumer, z. B. Silikonöl, Konservierungsmittel, Viskositätsregulatoren, Bindemittel und/oder Haftmittel, sowie Dünger oder andere Wirkstoffe zur Erzielung spezieller Effekte enthalten.

Die folgenden Beispiele dienen der Erläuterung der Erfindung. Sie schränken die Erfindung nicht ein. Temperaturen sind in Grad Celsius angegeben.

### Herstellungsbeispiele

Beispiel H1: 4,4-Difluorbut-3-ensäure-[2-(4-phenoxyphenoxy)äthylester] (Tabelle 1, Verbindung Nr. 1.01). 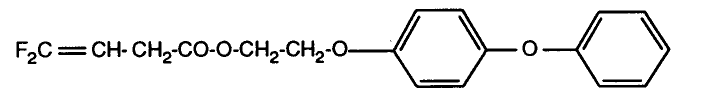
   Eine Lösung von 3,0 g 4,4-Difluorbut-3-ensäure in 50 ml Diäthyläther wird mit 0,36 g 4-Pyrrolidino-pyridin und 5,66 g 2-(4-Phenoxyphenoxy)äthanol versetzt und auf 0°C abgekühlt. Bei einer Temperatur zwischen 0°C und +5°C setzt man portionsweise insgesamt 5,58 g N,N'-Dicyclohexylcarbodiimid zu, entfernt die Eiskühlung und lässt das Reaktionsgemisch innerhalb von 16 Stunden unter Rühren auf Raumtemperatur erwärmen. Der als Niederschlag ausgefallene N,N'-Dicyclohexylharnstoff wird abgetrennt und verworfen. Die überstehende Lösung wird eingedampft. Der erhaltene Rückstand wird durch Säulenchromatographie an Kieselgel (Laufmittel: Hexan/Aethylacetat, 9:1) gereinigt. Man erhält so den reinen 4,4-Difluorbut-3-ensäure-[2-(4-phenoxyphenoxy)-äthylester] in Form eins Oels (Brechungsindex n_{D}²³: 1,5315).
Beispiel H2: In analoger Weise wie in Beispiel H1 beschrieben können auch die anderen in den folgenden Tabellen 1 bis 6 aufgeführten Verbindungen der Formel I hergestellt werden. In der Spalte "phys. Daten" dieser Tabellen steht "Smp." für den Schmelzpunkt der betreffenden Verbindung und "n_{D}^{T}" für den Brechungsindex der betreffenden Verbindung bei der Temperatur T°C.

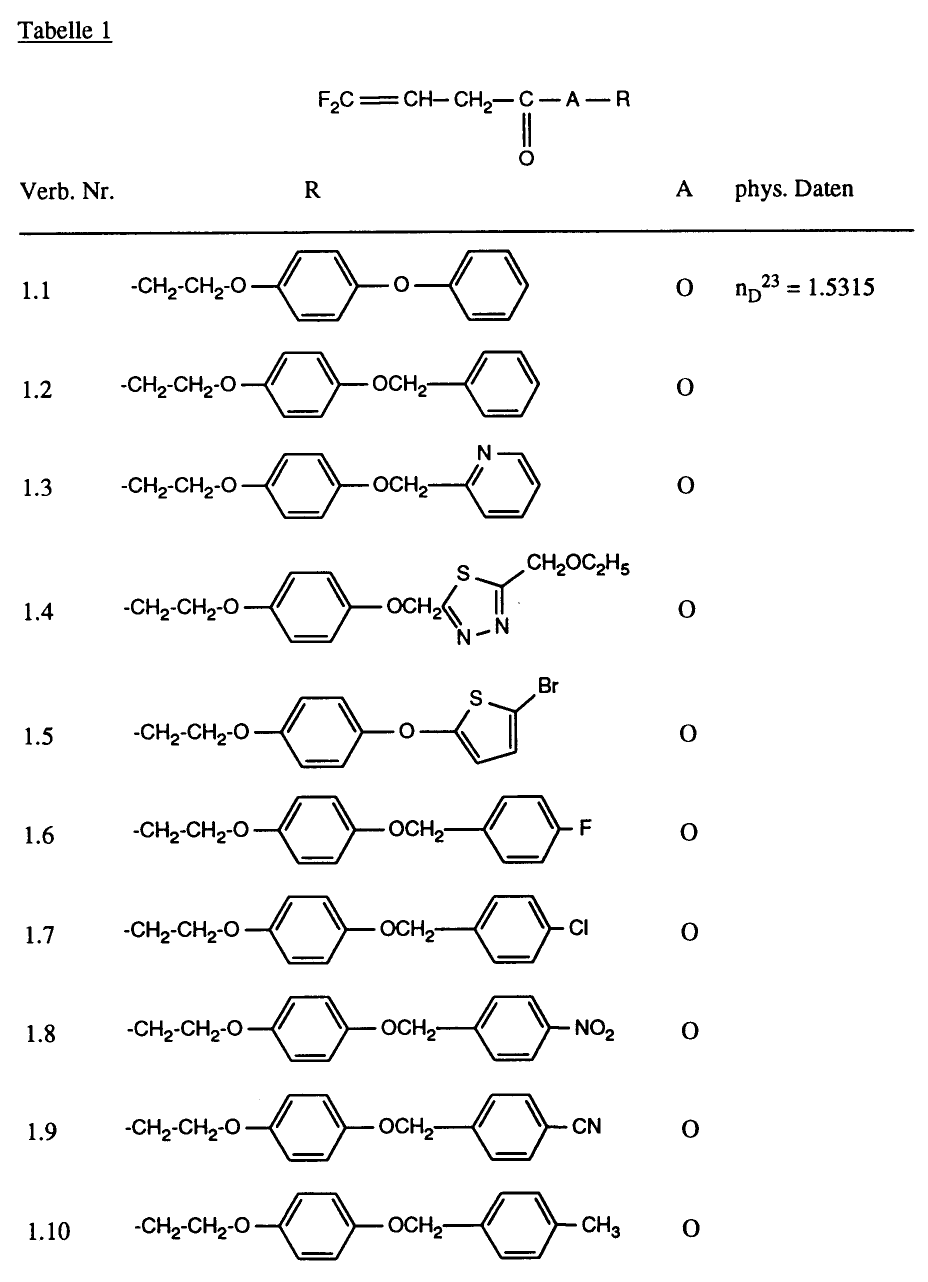

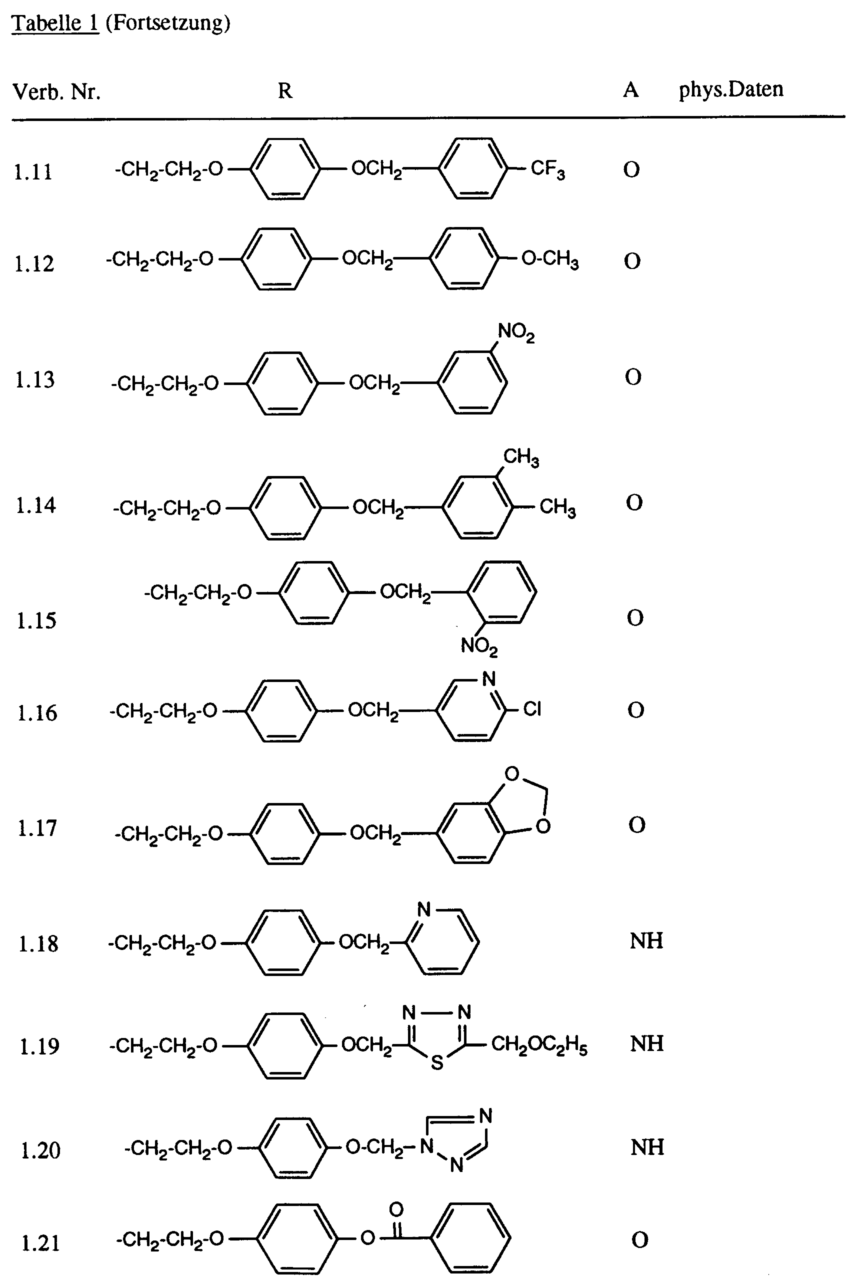

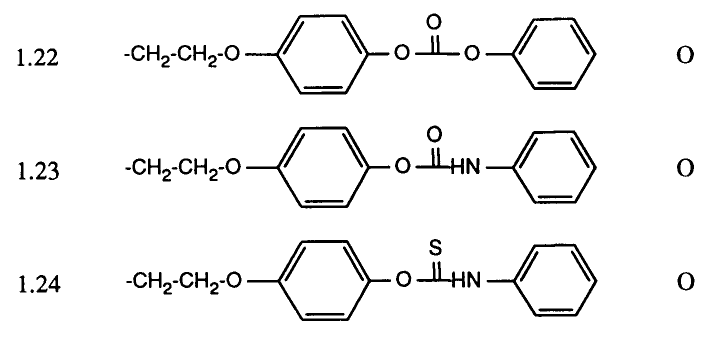

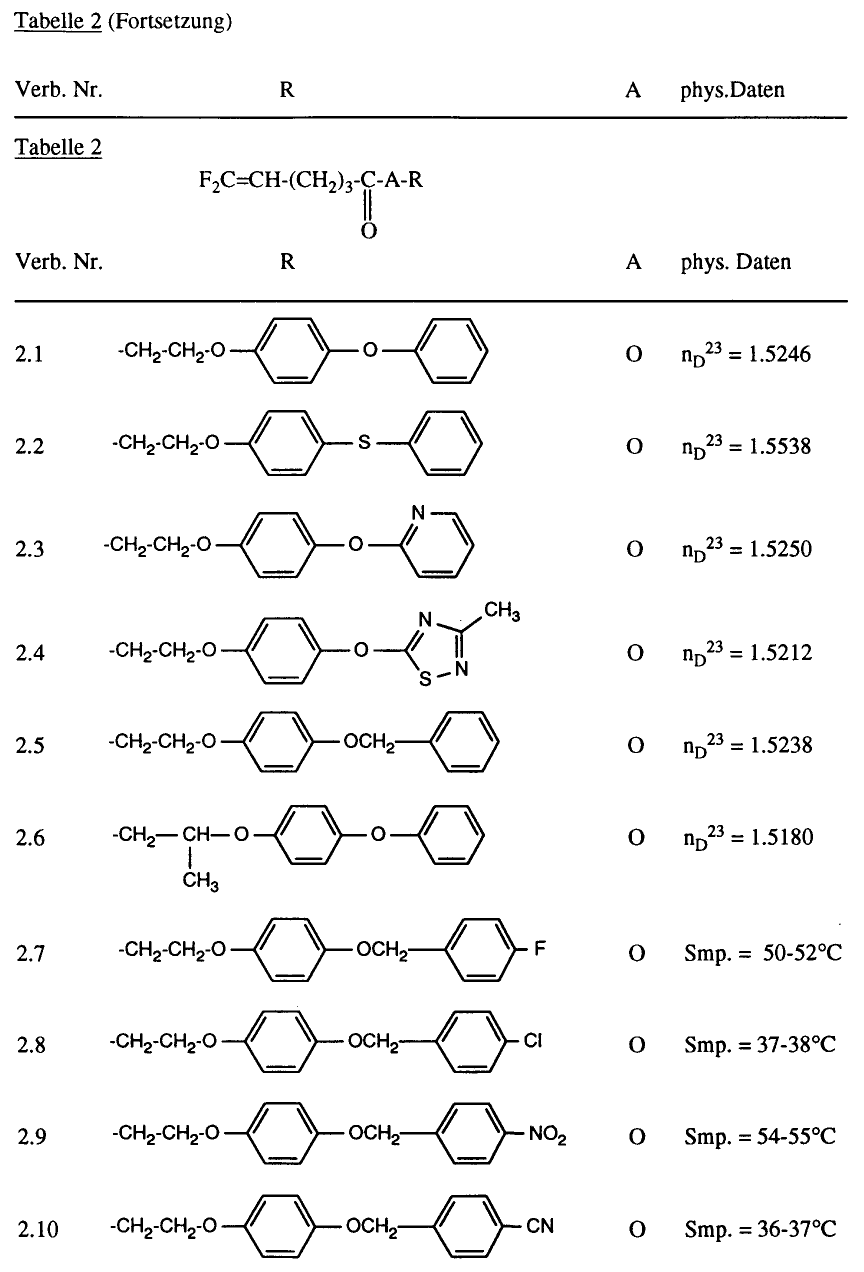

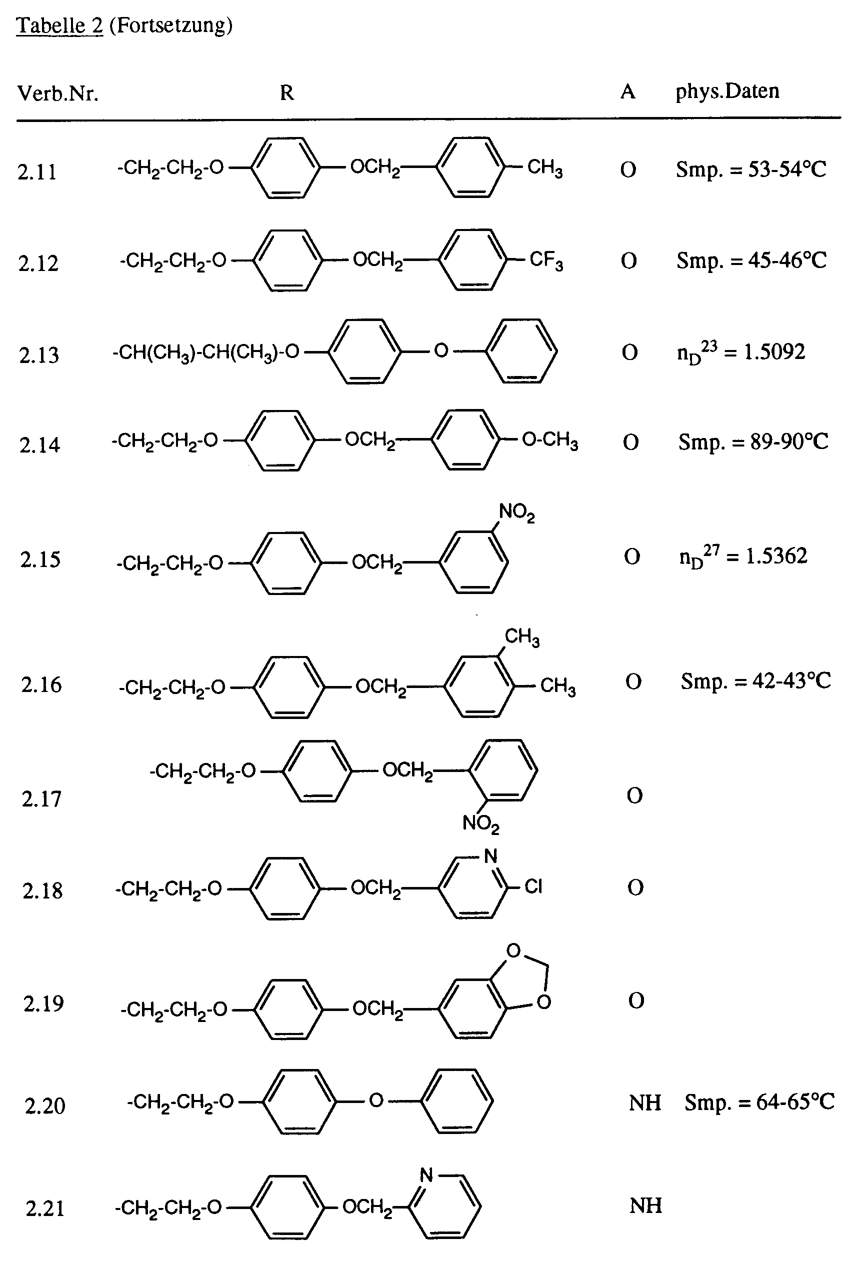

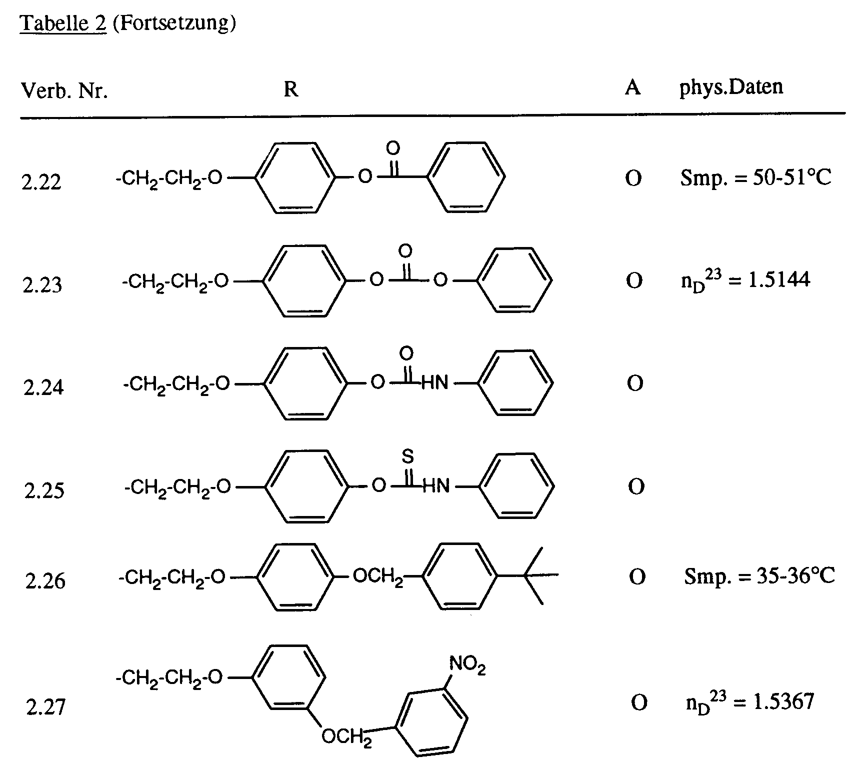

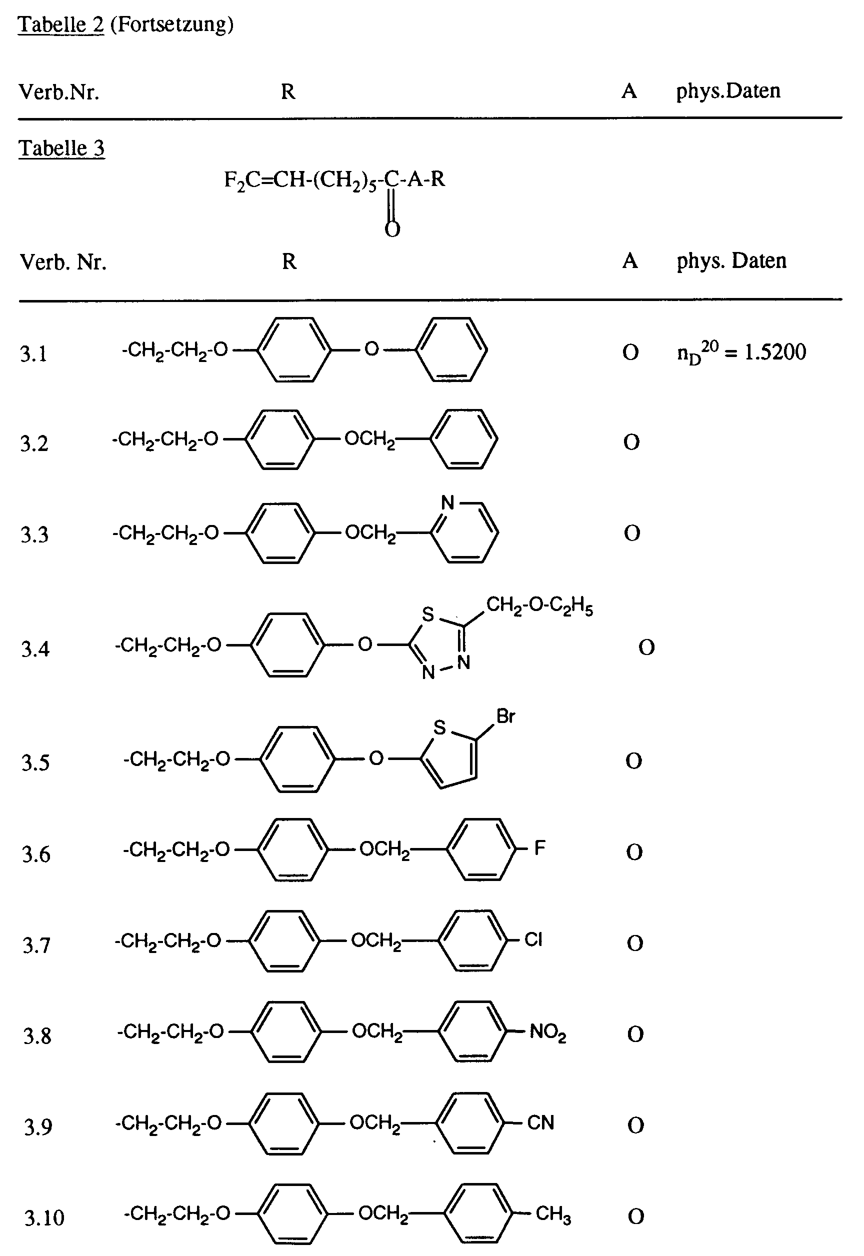

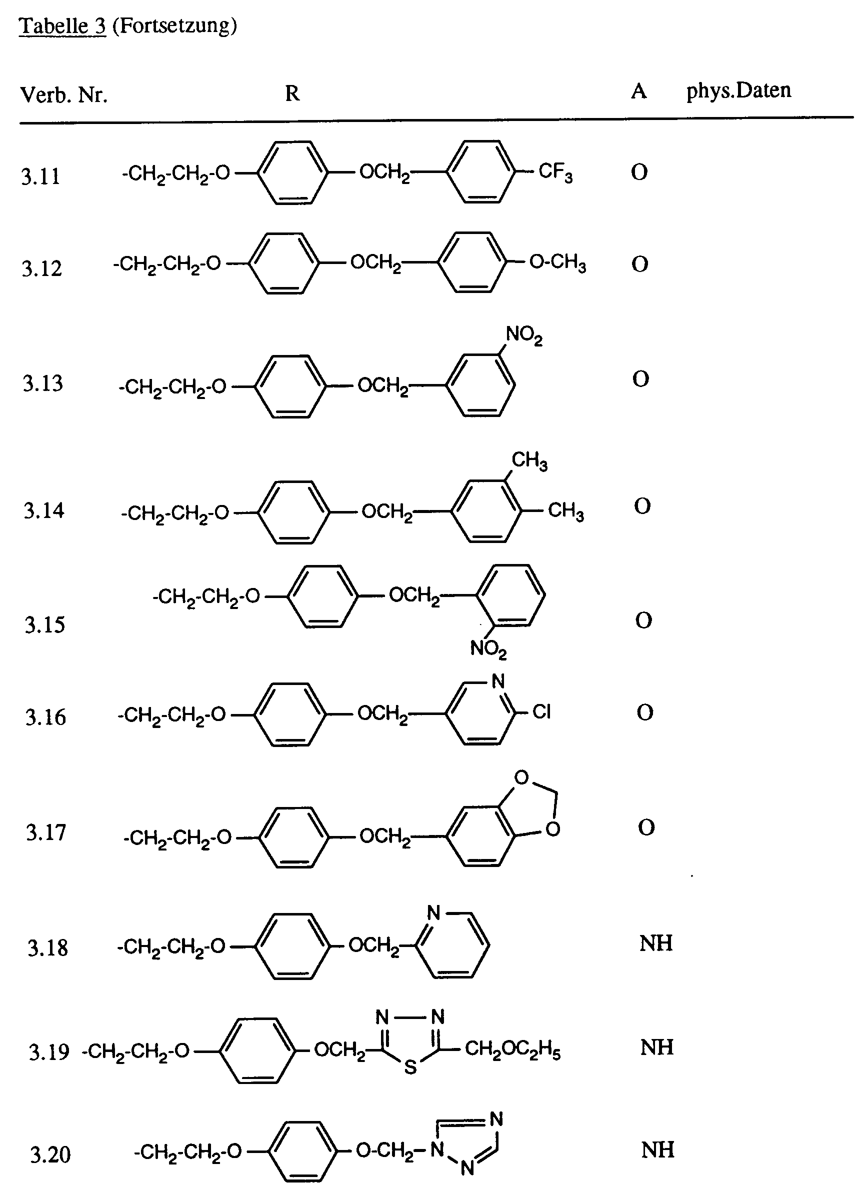

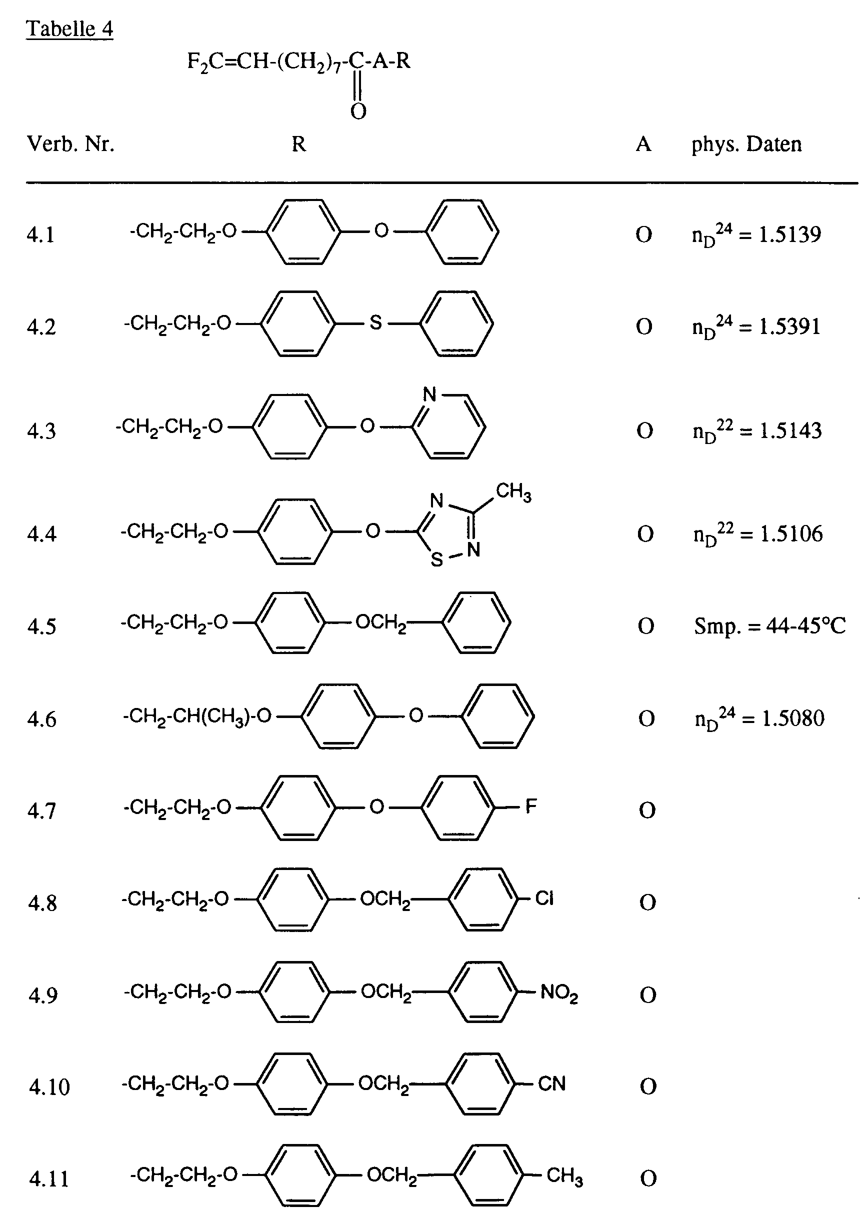

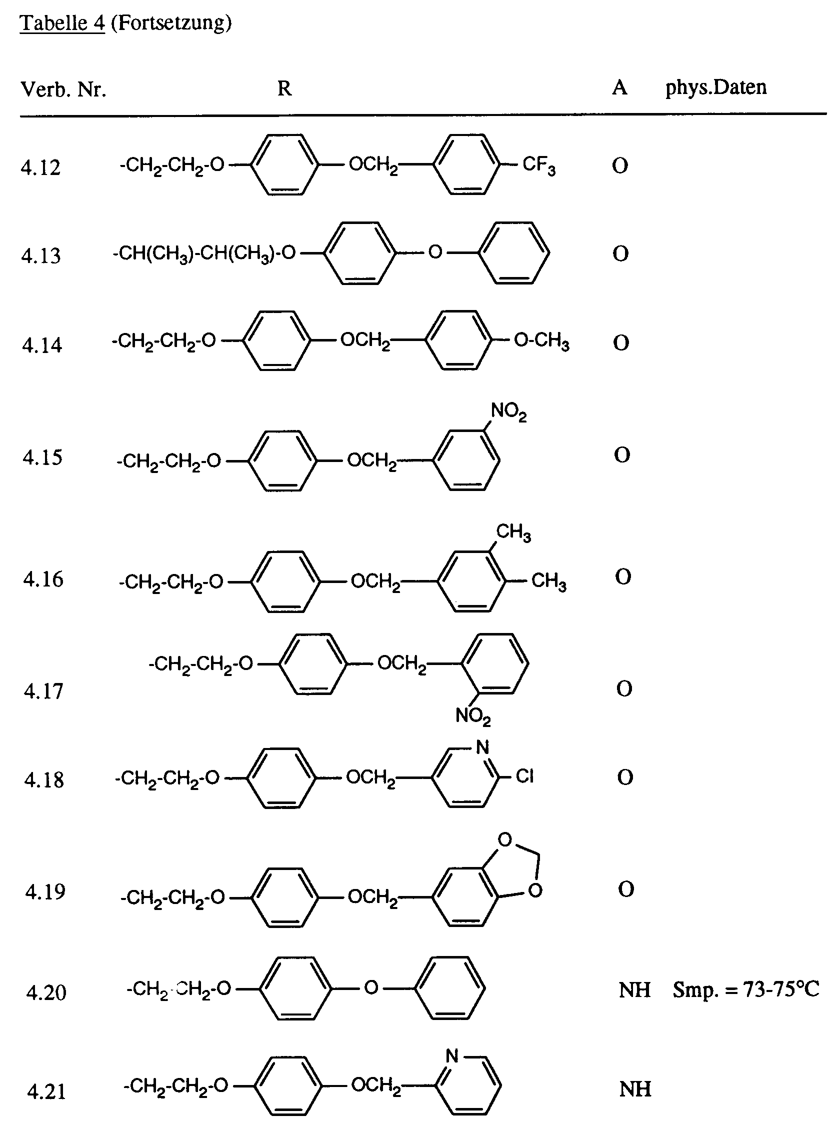

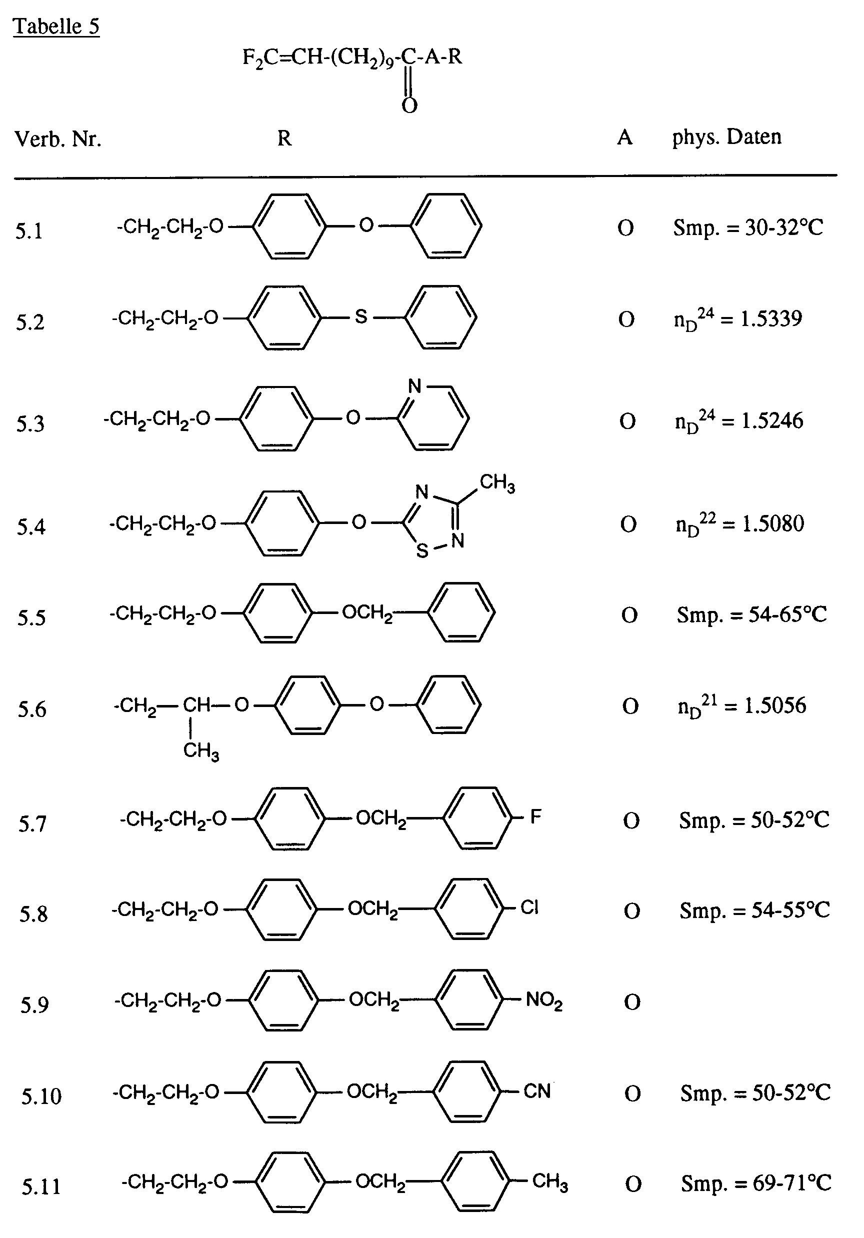

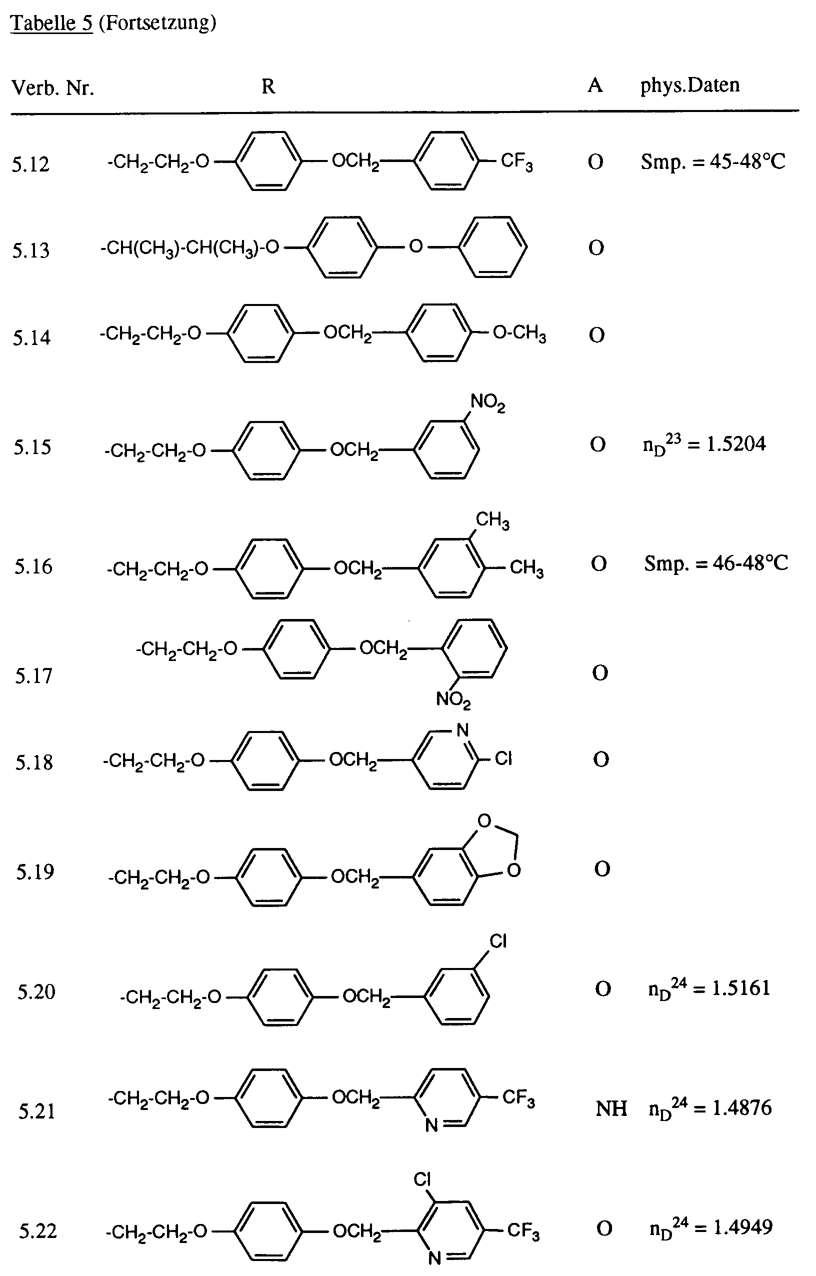

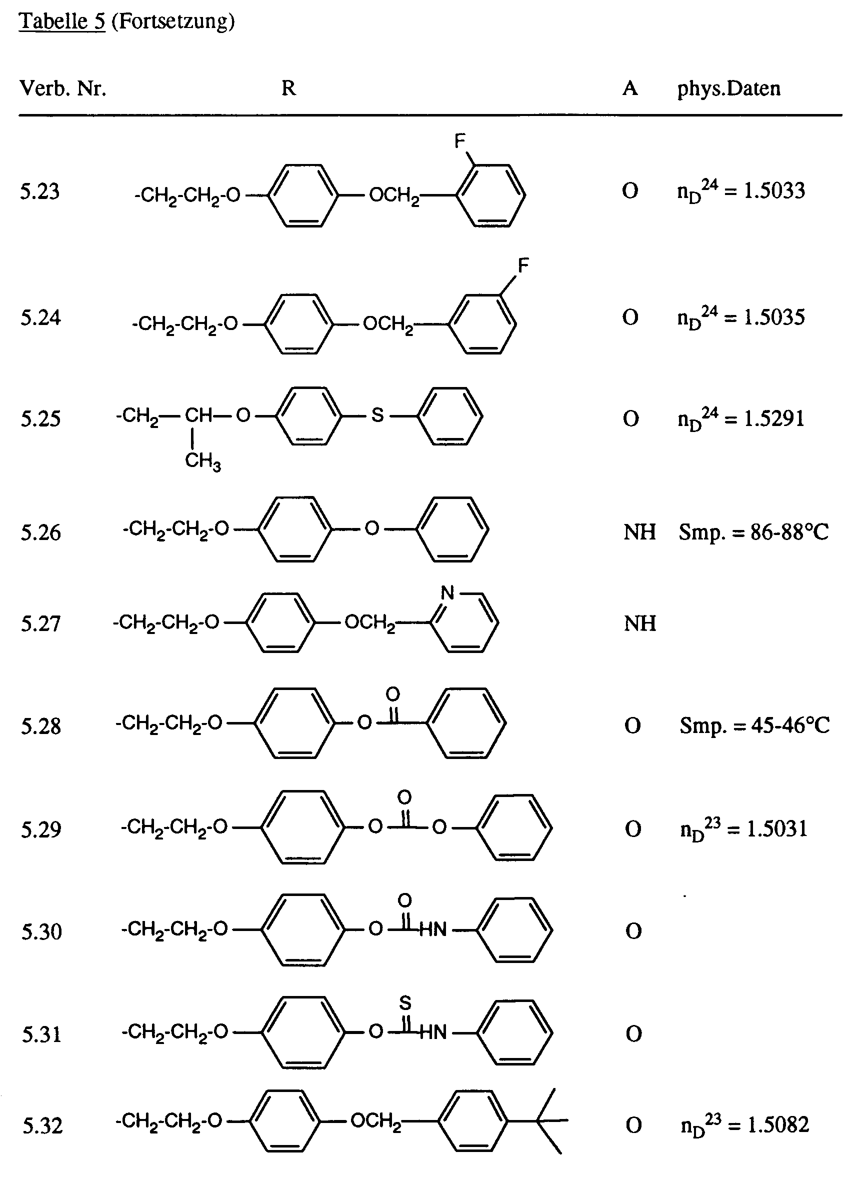

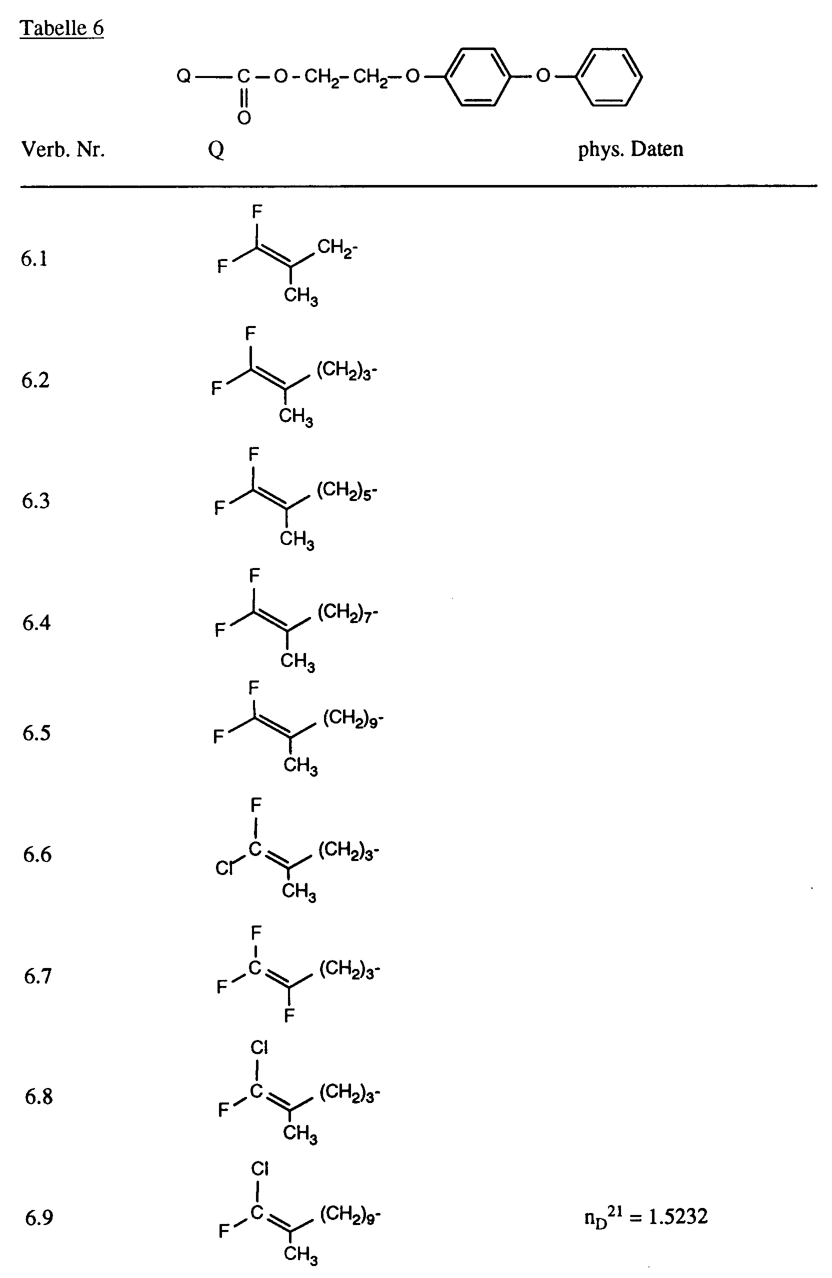

### Formulierungsbeispiele (% = Gewichtsprozent)

| Beispiel F1: Emulsions-Konzentrate | a) | b) | c) |
|---|---|---|---|
| Wirkstoff Nr. 1.1 | 25% | 40% | 50% |
| Ca-Dodecylbenzolsulfonat | 5% | 8% | 6% |
| Ricinusölpolyäthylenglykoläther (36 Mol AeO) | 5% | - | - |
| Tributylphenolpolyäthylenglykoläther (30 Mol AeO) | - | 12% | 4% |
| Cyclohexanon | - | 15% | 20% |
| Xylolgemisch | 65% | 25% | 20% |

Aus solchen Konzentraten können durch Verdünnen mit Wasser Emulsionen jeder gewünschten Konzentration hergestellt werden.

| Beispiel F2: Lösungen | a) | b) | c) | d) |
|---|---|---|---|---|
| Wirkstoff Nr. 1.1 | 80% | 10% | 5% | 95% |
| Aethylenglykolmonomethyläther | 20% | - | - | - |
| Polyäthylenglykol MG 400 | - | 70% | - | - |
| N-Methyl-2-pyrrolidon | - | 20% | - | - |
| Epoxidiertes Kokosnussöl | - | - | 1% | 5% |
| Benzin (Siedegrenzen 160-190°C) | - | - | 94% | - |

Die Lösungen sind zur Anwendung in Form kleinster Tropfen geeignet.

| Beispiel F3: Granulate | a) | b) | c) | d) |
|---|---|---|---|---|
| Wirkstoff Nr. 1.1 | 5% | 10% | 8% | 21% |
| Kaolin | 94% | - | 79% | 54% |
| Hochdisperse Kieselsäure | 1% | - | 13% | 7% |
| Attapulgit | - | 90% | - | 18% |

Der Wirkstoff wird in Methylenchlorid gelöst, auf den Träger aufgesprüht und das Lösungsmittel anschliessend im Vakuum abgedampft.

| Beispiel F4: Stäubemittel | a) | b) |
|---|---|---|
| Wirkstoff Nr. 1.1 | 2% | 5% |
| Hochdisperse Kieselsäure | 1% | 5% |
| Talkum | 97% | - |
| Kaolin | - | 90% |

Durch inniges Vermischen der Trägerstoffe mit dem Wirkstoff erhält man gebrauchsfertige Stäubemittel.

| Beispiel F5: Spritzpulver | a) | b) | c) |
|---|---|---|---|
| Wirkstoff Nr. 5.1 | 25% | 50% | 75% |
| Na-Ligninsulfonat | 5% | 5% | - |
| Na-Laurylsulfat | 3% | - | 5% |
| Na-Diisobutylnaphthalinsulfonat | - | 6% | 10% |
| Octylphenolpolyäthylenglykoläther (7-8 Mol AeO) | - | 2% | - |
| Hochdisperse Kieselsäure | 5% | 10% | 10% |
| Kaolin | 62% | 27% | - |

Der Wirkstoff wird mit den Zusatzstoffen vermischt und in einer geeigneten Mühle gut vermahlen. Man erhält Spritzpulver, die sich mit Wasser zu Suspensionen jeder gewünschten Konzentration verdünnen lassen.

| Beispiel F6: Emulsions-Konzentrat | |
|---|---|
| Wirkstoff Nr. 5.1 | 10% |
| Octylphenolpolyäthylenglykoläther (4-5 Mol AeO) | 3% |
| Ca-Dodecylbenzolsulfonat | 3% |
| Ricinusölpolyglykoläther (36 Mol AeO) | 4% |
| Cyclohexanon | 30% |
| Xylolgemisch | 50% |

Aus diesem Konzentrat können durch Verdünnen mit Wasser Emulsionen jeder gewünschten Konzentration hergestellt werden.

| Beispiel F7: Stäubemittel | a) | b) |
|---|---|---|
| Wirkstoff Nr. 5.1 | 5% | 8% |
| Talkum | 95% | - |
| Kaolin | - | 92% |

Man erhält anwendungsfertige Stäubemittel, indem der Wirkstoff mit dem Träger vermischt und auf einer geeigneten Mühle vermahlen wird.

| Beispiel F8: Extruder-Granulat | |
|---|---|
| Wirkstoff Nr. 5.1 | 10% |
| Na-Ligninsulfonat | 2% |
| Carboxymethylcellulose | 1% |
| Kaolin | 87% |

Der Wirkstoff wird mit den Zusatzstoffen vermischt, vermahlen und mit Wasser angefeuchtet. Dieses Gemisch wird extrudiert, granuliert und anschliessend im Luftstrom getrocknet.

| Beispiel F9: Umhüllungs-Granulat | |
|---|---|
| Wirkstoff Nr. 5.1 | 3% |
| Polyäthylenglykol (MG 200) | 3% |
| Kaolin | 94% |

Der fein gemahlene Wirkstoff wird in einem Mischer auf das mit Polyäthylenglykol angefeuchtete Kaolin gleichmässig aufgetragen. Auf diese Weise erhält man staubfreie Umhüllungs-Granulate.

| Beispiel F10: Suspensions-Konzentrat | |
|---|---|
| Wirkstoff Nr. 5.1 | 40% |
| Aethylenglykol | 10% |
| Nonylphenolpolyäthylenglykoläther (15 Mol AeO) | 6% |
| Na-Ligninsulfonat | 10% |
| Carboxymethylcellulose | 1% |
| Silikonöl in Form einer 75 %igen wässrigen Emulsion | 1% |
| Wasser | 32% |

Der fein gemahlene Wirkstoff wird mit den Zusatzstoffen innig vermischt. Man erhält so ein Suspensions-Konzentrat, aus welchem durch Verdünnen mit Wasser Suspensionen jeder gewünschten Konzentration hergestellt werden können.

### Biologische Beispiele

### Beispiel B1: Wirkung gegen Boophilus microplus

Vollgesogene adulte Zeckenweibchen werden auf eine PVC Platte aufgeklebt und mit einem Wattebausch überdeckt. Zur Behandlung werden die Testtiere mit 10 ml einer wässrigen Testlösung, die 125 ppm des zu prüfenden Wirkstoffes enthält übergossen. Anschliessend wird der Wattebausch entfernt und die Zecken werden für 4 Wochen zur Eiablage inkubiert. Die Wirkung gegen Boophilus microplus zeigt sich entweder beim Weibchen als Mortalität oder Sterilität, oder bei den Eiern als ovizide Wirkung.
Verbindungen der Tabellen 1 bis 6 zeigen in diesem Test gute Wirkung. Insbesondere zeigen die Verbindungen Nr. 1.1, 2.1 bis 2.6, 2.10, 2.12, 2.14 bis 2.16, 2.20, 2.22, 2.23, 2.26, 3.1, 4.1, 4.2, 4.5, 4.6, 4.20, 5.1 bis 5.3, 5.5, 5.6 und 5.20 bis 5.25 eine Wirkung von mehr als 80%.

### Beispiel B2: Wirkung gegen Nilaparvata lugens

Reispflanzen werden mit einer wässrigen Emulsions-Spritzbrühe, die 400 ppm des Wirkstoffes enthält, behandelt. Nach dem Antrocknen des Spritzbelages werden die Reispflanzen mit Zikadenlarven des 2. und 3. Stadiums besiedelt. 21 Tage später erfolgt die Auswertung. Aus dem Vergleich der Anzahl überlebender Zikaden auf den behandelten zu denjenigen auf den unbehandelten Pflanzen wird die prozentuale Reduktion der Population (% Wirkung) bestimmt.
Verbindungen der Tabellen 1 bis 6 zeigen in diesem Test gute Wirkung. Insbesondere zeigen die Verbindungen Nr. 1.1, 2.1 bis 2.3, 2.7, 2.8, 2.10 bis 2.16, 2.20, 2.22, 2.23, 2.26, 3.1, 4.1 bis 4.6, 4.20, 5.1 bis 5.6, 5.8, 5.10 bis 5.12, 5.15, 5.16 und 5.20 bis 5.25 eine Wirkung von mehr als 80%.

### Beispiel B3: Wirkung gegen Ctenocephalides felis

20 bis 25 Floheier werden in eine waagrecht stehend 50-ml-Zellkulturflasche gegeben, in der zuvor 15 g Flohlarven-Nährmedium, welches 100 ppm des zu prüfenden Wirkstoffs enthält, vorgelegt wurde. Die Testflaschen werden in einem Brutschrank bei 26 bis 27°C und 60-70 % Luftfeuchtigkeit bebrütet. Nach 21 Tagen wird die Anwesenheit von adulten Flöhen, nicht geschlüpften Puppen und Larven überprüft.
Verbindungen der Tabellen 1 bis 6 zeigen in diesem Test gute Wirkung. Insbesondere zeigen die Verbindungen Nr. 1.1, 2.2 bis 2.8, 2.10 bis 2.15, 2.22, 2.23, 2.26, 3.1, 4.3, 4.4, 5.8 und 5.10 bis 5.12 eine Wirkung von mehr als 80%.

### Beispiel B4: Wirkung gegen Aphis craccivora

Erbsenkeimlinge werden mit Aphis craccivora infiziert und anschliessend mit einer Spritzbrühe, die 400 ppm des Wirkstoffes enthält, besprüht und bei 20°C inkubiert. 3 und 6 Tage später erfolgt die Auswertung. Aus dem Vergleich der Anzahl toter Blattläuse auf den behandelten zu denjenigen auf den unbehandelten Pflanzen wird die prozentuale Reduktion der Population (% Wirkung) bestimmt.
Die Verbindungen der Tabellen 1 bis 6 zeigen in diesem Test gute Wirkung. Insbesondere zeigen die Verbindungen Nr. 1.1, 2.1 bis 2.7, 2.10 bis 2.13, 2.22, 2.23, 3.1, 5.5 und 5.20 bis 5.25 eine Wirkung von mehr als 80%.

### Beispiel B5: Systemische Wirkung gegen Nilaparvata lugens

Töpfe mit Reispflanzen werden in eine wässrige EmulsionsLösung, die 400 ppm des Wirkstoffes enthält, gestellt. Anschliessend werden die Reispflanzen mit Larven des 2. und 3. Stadiums besiedelt. 6 Tage später erfolgt die Auswertung. Aus dem Vergleich der Anzahl Zikaden auf den behandelten zu denjenigen auf den unbehandelten Pflanzen wird die prozentuale Reduktion der Population (% Wirkung) bestimmt.
Die Verbindungen der Tabellen 1 bis 6 zeigen in diesem Test gute Wirkung. Insbesondere zeigen die Verbindungen Nr. 1.1, 2.1 bis 2.8, 2.10 bis 2.16, 2.20, 2.22, 2.23, 2.26, 3.1, 4.1 bis 4.6, 4.20, 5.1 bis 5.8, 5.10 bis 5.12, 5.15, 5.16 und 5.20 bis 5.25 eine Wirkung von mehr als 80%.

### Beispiel B6: Wirkung gegen Panonychus ulmi (OP und Carb. resistent)

Apfelsämlinge werden mit adulten Weibchen von Panonychus ulmi besiedelt. Nach sieben Tagen werden die infestierten Pflanzen mit einer wässrigen Emulsion enthaltend 400 ppm der zu prüfenden Verbindung bis zur Tropfennässe besprüht und im Gewächshaus kultiviert. Nach 14 Tagen erfolgt die Auswertung. Aus dem Vergleich der Anzahl toter Spinnmilben auf den behandelten zu denjenigen auf den unbehandelten Pflanzen wird die prozentuale Reduktion an Population (% Wirkung) bestimmt.
Die Verbindungen der Tabellen 1 bis 6 zeigen in diesem Test gute Wirkung. Insbesondere zeigen die Verbindungen Nr. 1.1, 2.1, 2.7, 2.8, 2.10, 2.12 bis 2.16, 2.22, 2.23, 2.26, 3.1, 4.1, 4.2, 4.6, 5.1 bis 5.3, 5.5, 5.6, 5.8, 5.10, 5.11, 5.15, 5.16 und 5.20 bis 5.25 eine Wirkung von mehr als 80%.

### Beispiel B7: Wirkung gegen Nephotettix cincticeps

Reispflanzen werden mit einer wässrigen Emulsions-Spritzbrühe, die 400 ppm des Wirkstoffes enthält, behandelt. Nach dem Antrocknen des Spritzbelages werden die Reispflanzen mit Zikadenlarven des 2. und 3. Stadiums besiedelt. 21 Tage später erfolgt die Auswertung. Aus dem Vergleich der Anzahl überlebender Zikaden auf den behandelten zu denjenigen auf den unbehandelten Pflanzen wird die prozentuale Reduktion der Population (% Wirkung) bestimmt.
Die Verbindungen der Tabellen 1 bis 6 zeigen in diesem Test gute Wirkung. Insbesondere zeigen die Verbindungen Nr. 1.1, 2.1, 2.2, 2.3, 2.6, 2.7, 2.10 bis 2.12, 2.14 bis 2.16, 2.23, 2.26, 3.1, 4.1, 4.2, 4.5, 4.6, 5.1 bis 5.3, 5.5, 5.10 bis 5.12, 5.15, 5.16, 5.20, 5.22, 5.24 und 5.25 eine Wirkung von mehr als 80%.

### Beispiel B8: Wirkung gegen Bemisia tabaci

Buschbohnen-Pflanzen werden in Gazekäfige gestellt und mit Adulten von Bemisia tabaci (Weisse Fliege) besiedelt. Nach erfolgter Eiablage werden alle Adulten entfernt und 10 Tage später die Pflanzen mit den darauf befindenden Nymphen mit einer wässrigen Emulsions-Spritzbrühe der zu prüfenden Wirkstoffe (Konzentration 400 ppm) behandelt. Die Auswertung erfolgt 14 Tage nach der Wirkstoff-Applikation auf %-Schlupf im Vergleich zu den unbehandelten Kontrollansätzen.
Die Verbindungen der Tabellen 1 bis 6 zeigen in diesem Test gute Wirkung. Insbesondere zeigen die Verbindungen Nr. 1.1, 2.1 bis 2.8, 2.10 bis 2.16, 2.22, 2.23, 3.1, 4.1, 4.2, 4.5, 4.6, 5.2, 5.3, 5.6 bis 5.8, 5.10, 5.11, 5.20, 5.21 und 6.9 eine Wirkung von mehr als 80%.

### Beispiel B9: Wirkung gegen Tetranychus urticae

Junge Bohnenpflanzen werden mit einer Mischpopulation von Tetranychus urticae besiedelt und 1 Tag später mit einer wässrigen Emulsions-Spritzbrühe, die 400 ppm des Wirkstoffes enthält, besprüht. Die Pflanzen werden anschliessend für 6 Tage bei 25°C inkubiert und danach ausgewertet. Aus dem Vergleich der Anzahl toter Eier, Larven und Adulten auf den behandelten zu denjenigen auf den unbehandelten Pflanzen wird die prozentuale Reduktion der Population (% Wirkung) bestimmt.
Die Verbindungen der Tabellen 1 bis 6 zeigen in diesem Test gute Wirkung. Insbesondere zeigen die Verbindungen Nr. 1.1, 2.1 bis 2.8, 2.10, 2,12 bis 2.16, 2.22, 2.23, 2.26, 3.1, 4.1 bis 4.6, 4.20, 5.1 bis 5.6, 5.8, 5.10 bis 5.12, 5.15, 5.16 und 5.20 bis 5.25 eine Wirkung von mehr als 80%.

### Beispiel B10: Wirkung gegen Heliothis virescens Raupen

Junge Sojapflanzen werden mit einer wässrigen Emulsions-Spritzbrühe, die 400 ppm des Wirkstoffes enthält, besprüht. Nach dem Antrocknen des Spritzbelages werden die Sojapflanzen mit 10 Raupen des ersten Stadiums von Heliothis virescens besiedelt und in einen Plastikbehälter gegeben. 6 Tage später erfolgt die Auswertung. Aus dem Vergleich der Anzahl toter Raupen und des Frasschadens auf den behandelten zu denjenigen auf den unbehandelten Pflanzen wird die prozentuale Reduktion der Population bzw. die prozentuale Reduktion des Frassschadens (% Wirkung) bestimmt.
Die Verbindungen der Tabellen 1 bis 6 zeigen in diesem Test gute Wirkung. Insbesondere zeigen die Verbindungen Nr. 1.1, 2.1 bis 2.8, 2.10 bis 2.16, 2.22, 2.23, 2.26, 3.1, 4.1, 4.2, 4.5, 4.6, 4.20, 5.1 bis 5.3, 5.5 bis 5.8, 5.10 bis 5.12, 5.15, 5.16, 5.20 bis 5.22, 5.24 und 5.25 eine Wirkung von mehr als 80%.

### Beispiel B11: Wirkung gegen Crocidolomia binotalis Raupen

Junge Kohlpflanzen werden mit einer wässrigen Emulsions-Spritzbrühe, die 400 ppm des Wirkstoffes enthält, besprüht. Nach dem Antrocknen des Spritzbelages werden die Kohlpflanzen mit 10 Raupen des dritten Stadiums von Crocidolomia binotalis besiedelt und in einen Plastikbehälter gegeben. 3 Tage später erfolgt die Auswertung. Aus dem Vergleich der Anzahl toter Raupen und des Frasschadens auf den behandelten zu denjenigen auf den unbehandelten Pflanzen wird die prozentuale Reduktion der Population bzw. die prozentuale Reduktion des Frasschadens (% Wirkung) bestimmt.
Die Verbindungen der Tabellen 1 bis 6 zeigen in diesem Test gute Wirkung. Insbesondere zeigen die Verbindungen Nr. 1.1, 3.1, 5.1, 5.2, 5.5 und 5.20 bis 5.25 eine Wirkung von mehr als 80%.

### Beispiel B12: Wirkung gegen Anthonomus grandis Adulte

Junge Baumwollpflanzen werden mit einer wässrigen Emulsions-Spritzbrühe, die 400 ppm des Wirkstoffes enthält, besprüht. Nach dem Antrocknen des Spritzbelages werden die Baumwollpflanzen mit 10 Adulten von Anthonomus grandis besiedelt und in einen Plastikbehälter gegeben. 3 Tage später erfolgt die Auswertung. Aus dem Vergleich der Anzahl toter Käfer und des Frasschadens auf den behandelten zu denjenigen auf den unbehandelten Pflanzen wird die prozentuale Reduktion der Population bzw. die prozentuale Reduktion des Frasschadens (% Wirkung) bestimmt.
Die Verbindungen der Tabellen 1 bis 6 zeigen in diesem Test gute Wirkung. Insbesondere zeigen die Verbindungen Nr. 1.1, 2.7, 2.8, 2.10 bis 2.16, 3.1, 5.1, 5.2, 5.5, 5.10, 5.16 und 5.20 bis 5.25 eine Wirkung von mehr als 80%.

### Beispiel B13: Systemische Wirkung gegen Myzus persicae

Erbsenkeimlinge werden mit Myzus persicae infiziert, anschliessend mit den Wurzeln in eine Spritzbrühe, die 400 ppm des Wirkstoffes enthält, gestellt und bei 20°C inkubiert. 3 und 6 Tage später erfolgt die Auswertung. Aus dem Vergleich der Anzahl toter Blattläuse auf den behandelten zu denjenigen auf den unbehandelten Pflanzen wird die prozentuale Reduktion der Population (% Wirkung) bestimmt.
Die Verbindungen der Tabellen 1 bis 6 zeigen in diesem Test gute Wirkung. Insbesondere zeigt die Verbindung Nr. 1.1 eine Wirkung von mehr als 80%.

### Beispiel B14: Systemische Wirkung gegen Nephotettix cincticeps

Töpfe mit Reispflanzen werden in eine wässrige Emulsions-Lösung, die 400 ppm des Wirkstoffes enthält, gestellt. Anschliessend werden die Reispflanzen mit Larven des 2. und 3. Stadiums besiedelt. 6 Tage später erfolgt die Auswertung. Aus dem Vergleich der Anzahl Zikaden auf den behandelten zu denjenigen auf den unbehandelten Pflanzen wird die prozentuale Reduktion der Population (% Wirkung) bestimmt.
Die Verbindungen der Tabellen 1 bis 6 zeigen in diesem Test gute Wirkung. Insbesondere zeigen die Verbindungen Nr. 1.1, 2.1 bis 2.6, 3.1, 4.1, 4.2, 4.6, 5.1 bis 5.3 und 5.24 eine Wirkung von mehr als 80%.

### Beispiel B15: Ovo/larvizide Wirkung auf Heliothis virescens

Auf Baumwolle abgelegte Eier von Heliothis werden mit einer wässrigen Emulsions-Spritzbrühe, die 400 ppm des Wirkstoffes enthält, besprüht. Nach 8 Tagen wird der prozentuale Schlupf der Eier und die Ueberlebensrate der Raupen im Vergleich zu unbehandelten Kontrollansätzen ausgewertet (% Reduktion der Population).
Die Verbindungen der Tabellen 1 bis 6 zeigen in diesem Test gute Wirkung. Insbesondere zeigen die Verbindungen Nr. 1.1, 2.1 bis 2.7, 2.10 bis 2.12, 2.15, 2.16, 2.22, 2.23, 2.26, 3.1, 4.1 bis 4.6, 4.20, 5.1 bis 5.8, 5.10 bis 5.12, 5.15, 5.16, 5.20 bis 5.26 und 6.9 eine Wirkung von mehr als 80%.

### Beispiel B16: Wirkung gegen Dermanyssus gallinae

In einen nach oben offenen Glasbehälter werden 2 bis 3 ml einer 10 ppm Wirkstoff enthaltenden Lösung und ca. 200 Milben in unterschiedlichen Entwicklungsstadien gegeben. Anschliessend wird der Behälter mit einem Wattebausch verschlossen, 10 Minuten lang bis zur vollständigen Benetzung der Milben geschüttelt und dann kurzfristig umgekehrt, damit die restliche Testlösung von der Watte aufgenommen werden kann. Nach 3 Tagen wird die Mortalität der Milben durch Auszählen der toten Individuen ermittelt und in Prozent angegeben.
Die Verbindungen der Tabellen 1 bis 6 zeigen in diesem Test gute Wirkung. Insbesondere zeigen die Verbindungen Nr. 1.1, 2.6, 2.13, 4.1, 4.2, 4.4, 4.5, 4.6 und 5.21 eine Wirkung von mehr als 80%.

## Patentansprüche

1. Eine Verbindung der Formel 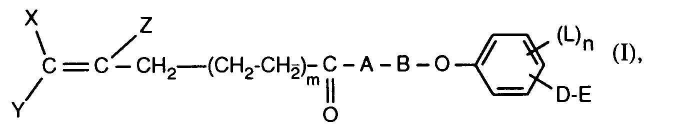 worin
A Sauerstoff, Schwefel oder-NR₁-;
B C₂-C₆-Alkylen,
D-E -O-E, -S-E, -O-CH₂-E, -O-C(=O)-E, -O-C(=O)-O-E, -O-C(=O)-N(H)-E oder -O-C(=S)-N(H)-E;
E Phenyl; durch ein bis drei Substituenten, ausgewählt aus der Gruppe, bestehend aus Halogen, C₁-C₄-Alkyl, Halogen-C₁-C₄-alkyl, C₁-C₄-Alkoxy, Halogen-C₁-C₄-alkoxy, Cyano, Nitro und Methylendioxy, substituiertes Phenyl; einen fünfgliedrigen aromatischen Heterocyclus mit ein bis drei Heteroatomen, ausgewählt aus der Gruppe, bestehend aus Stickstoff, Sauerstoff und Schwefel; einen durch ein oder zwei Substituenten, ausgewählt aus der Gruppe, bestehend aus Halogen, C₁-C₄-Alkyl und C₁-C₄-Halogenalkyl, substituierten fünfgliedrigen aromatischen Heterocyclus mit ein bis drei Heteroatomen, ausgewählt aus der Gruppe, bestehend aus Stickstoff, Sauerstoff und Schwefel; einen sechsgliedrigen aromatischen Heterocyclus mit ein bis drei Stickstoffatomen; oder einen durch ein oder zwei Substituenten, ausgewählt aus der Gruppe, bestehend aus Halogen, C₁-C₄-Alkyl und C₁-C₄-Halogenalkyl, substituierten sechsgliedrigen aromatischen Heterocyclus mit ein bis drei Stickstoffatomen;
L Halogen oder Methyl;
X Fluor;
Y Chlor oder Fluor;
Z Wasserstoff, Fluor oder Methyl;
m die Zahl null, eins, zwei, drei, vier oder fünf;
n die Zahl null, eins oder zwei und
R₁ Wasserstoff, C₁-C₄-Alkyl, Phenylthio oder Tolylthio bedeuten, in freier Form oder gegebenenfalls in Salzform.

2. Eine Verbindung gemäss Anspruch 1 der Formel I, worin A für Sauerstoff steht.

3. Eine Verbindung gemäss Anspruch 1 oder 2 der Formel I, worin B für eine Aethylenbrücke steht.

4. Eine Verbindung gemäss Anspruch 1 der Formel I, worin A für eine Brücke - NH- steht.

5. Eine Verbindung gemäss einem der Ansprüche 1, 3 und 4 der Formel I, worin n für null steht.

6. Eine Verbindung gemäss einem der Ansprüche 1 bis 5 der Formel I, worin D für - O- steht.

7. Eine Verbindung gemäss einem der Ansprüche 1 bis 6 der Formel I, worin E für Phenyl; Pyridyl; Thiadiazolyl; durch ein oder zwei Substituenten, ausgewählt aus der Gruppe, bestehend aus Cyano, Nitro, Halogen, C₁-C₄-Alkyl und C₁-C₄-Halogenalkyl, substituiertes Phenyl; durch ein oder zwei Substituenten, ausgewählt aus der Gruppe, bestehend aus Halogen, C1-C4-Alkyl und Halogen-C₁-C₄-alkyl, substituiertes Pyridyl; oder C₁-C₄-Alkylthiazolyl steht.

8. Eine Verbindung gemass einem der Ansprüche 1 bis 7 der Formel I, worin m für null, eins oder vier steht.

9. Eine Verbindung gemass einem der Ansprüche 1 bis 8 der Formel I, worin Y für Fluor und Z für Wasserstoff stehen.

10. Eine Verbindung gemäss einem der Ansprüche 1, 3 bis 5 und 7 bis 9 der Formel I, worin D für -O-CH₂- steht.

11. Eine Verbindung gemäss einem der Ansprüche 1 bis 5 und 7 bis 9 der Formel I, worin D für -O-CO- oder -O-CO-O- steht.

12. Eine Verbindung gemäss Anspruch 2 der Formel I, worin n null ist.

13. Eine Verbindung gemäss Anspruch 12 der Formel I, worin D Sauerstoff oder Schwefel bedeutet.

14. Eine Verbindung gemäss Anspruch 2 der Formel I, worin D -O-CH₂- bedeutet.

15. Eine Verbindung gemäss Anspruch 6 der Formel I, worin E für eine gegebenenfalls substituierte Phenyl-, Thiadiazolyl- oder Pyridyl-Gruppe steht.

16. Eine Verbindung gemäss Anspruch 3 der Formel I, worin A für Sauerstoff, n für null, D für Sauerstoff, Schwefel oder -O-CH₂-, E für Phenyl; Pyridyl; Thiadiazolyl; durch ein oder zwei Substituenten, ausgewählt aus der Gruppe, bestehend aus Cyano, Nitro, Halogen, C₁-C₄-Alkyl und C₁-C₄-Halogenalkyl, substituiertes Phenyl; durch ein oder zwei Substituenten, ausgewählt aus der Gruppe, bestehend aus Halogen, C₁-C₄-Alkyl und Halogen-C₁-C₄-alkyl, substituiertes Pyridyl; oder C₁-C₄-Alkylthiadiazolyl, m für null, eins oder vier, Y für Fluor und Z für Wasserstoff steht.

17. Eine Verbindung gemäss Anspruch 16 der Formel I, worin m 1 ist, B -CH₂CH₂- ist, D-E -O-CH₂-E ist und E 4-Trifluormethylphenyl ist.

18. Eine Verbindung gemäss Anspruch 1 der Formel I, ausgewählt aus der Gruppe, bestehend aus den Verbindungen
4,4-Difluorbut-3-ensäure-[2-(4-phenoxyphenoxy)äthylester],
4,4-Difluorbut-3-ensäure-[2-(4-benzyloxyphenoxy)äthylester],
4,4-Difluorbut-3-ensäure-[2-(4-{4-methyl}-benzyloxyphenoxy)äthylester],
6,6-Difluorhex-5-ensäure-[2-(4-phenoxyphenoxy)äthylester],
6,6-Difluorhex-5-ensäure-[2-(4-benzyloxyphenoxy)äthylester],
8,8-Difluoroct-7-ensäure-[2-(4-phenoxyphenoxy)äthylester],
8,8-Difluoroct-7-ensäure-[2-(4-benzyloxyphenoxy)äthylester],
10,10-Difluordec-9-ensäure-[2-(4-phenoxyphenoxy)äthylester],
10,10-Difluordec-9-ensäure-[2-(4-benzyloxyphenoxy)äthylester],
12,12-Difluordodec-11-ensäure-[2-(4-phenoxyphenoxy)äthylester], und
12,12-Difluordodec-11-ensäure-[2-(4-benzyloxyphenoxy)äthylester].

19. 4,4-Difluorbut-3-ensäure-[2-(4-{4-trifluormethyl}benzyloxyphenoxy)äthylester] gemäss Anspruch 1.

20. 6,6-Difluorhex-5-ensäure-[2-(4-{4-trifluormethyl}benzyloxyphenoxy)äthylester] gemäss Anspruch 1.

21. 6,6-Difluorhex-5-ensäure-[2-(4-{4-tert-butyl}benzyloxyphenoxy)äthylester] gemäss Anspruch 1.

22. 12,12-Difluordodec-11-ensäure-[2-(4-{4-trifluormethyl}benzyloxyphenoxy)äthylester] gemäss Anspruch 1.

23. 12,12-Difluordodec-11-ensäure-[2-(4-{4-tert.butyl}benzyloxyphenoxy)äthylester] gemäss Anspruch 1.

24. Verfahren zur Herstellung einer Verbindung gemäss Anspruch 1 der Formel I, dadurch gekennzeichnet, dass man
a) eine Verbindung der Formel 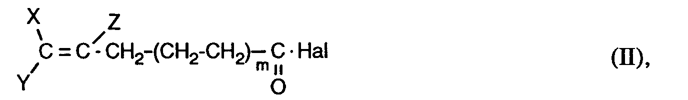 worin X, Y, Z und m die für die Formel I angegebenen Bedeutungen haben und Hal für Chlor oder Brom steht, gegebenenfalls in einem inerten Lösungsmittel und in Gegenwart eines säurebindenden Mittels, mit einer Verbindung der Formel 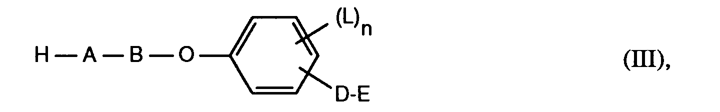 worin A, B, L, D, E und n die für die Formel I angegebenen Bedeutungen haben, oder gegebenenfalls einem Salz davon umsetzt oder
b) eine Verbindung der Formel 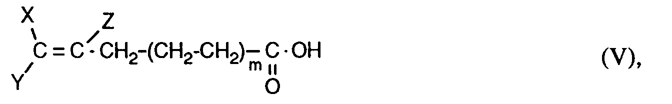 worin X, Y, Z und m die für die Formel I angegebenen Bedeutungen haben, gegebenenfalls in Gegenwart eines inerten Lösungsmittels und eines Katalysators oder eines wasserentziehenden Mittels, mit einer Verbindung der Formel III oder gegebenenfalls einem Salz davon umsetzt
und jeweils, wenn erwünscht, eine verfahrensgemäss erhältliche freie Verbindung der Formel I in ein Salz oder ein verfahrensgemäss erhältliches Salz einer Verbindung der Formel I in die freie Verbindung der Formel I oder in ein anderes Salz überführt.

25. Verfahren zur Herstellung einer Verbindung gemäss Anspruch 1 der Formel I, worin A Sauerstoff oder Schwefel bedeutet, dadurch gekennzeichnet, dass man eine Verbindung der Formel 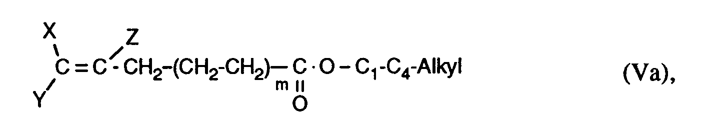 worin X, Y, Z und m die für die Formel I angegebenen Bedeutungen haben, basen- oder säurekatalysiert mit einer Verbindung der Formel 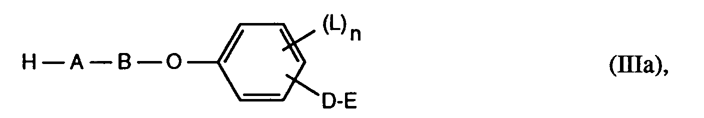 worin B, D, E, L und n die für die Formel I angegebenen Bedeutungen haben und A für Sauerstoff oder Schwefel steht, umsetzt.

26. Verfahren zur Herstellung einer Verbindung gemäss Anspruch 1 der Formel I, worin A NR₁ bedeutet, dadurch gekennzeichnet, dass man eine Verbindung der Formel (Va), wie in Anspruch 25 definiert, mit einer Verbindung der Formel 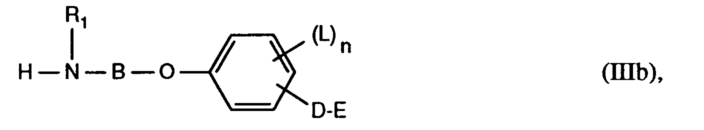 worin R₁, B, D, E, L und n die für die Formel I angegebenen Bedeutungen haben, oder einem Salz davon umsetzt.

27. Verfahren zur Herstellung einer Verbindung der Formel I, worin Z für Wasserstoff oder Methyl steht, in freier Form oder in Salzform, dadurch gekennzeichnet, dass man eine Verbindung der Formel 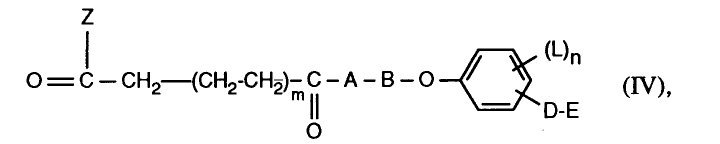 worin A, B, L, D, E, m und n die für die Formel I angegebenen Bedeutungen haben und Z für Wasserstoff oder Methyl steht, oder gegebenenfalls ein Salz davon in einem inerten Lösungsmittel, in Gegenwart eines trisubstituierten Phosphans, mit einem Halogenmethan oder einem Trihalogenessigsäure-Alkalisalz umsetzt.

28. Schädlingsbekämpfungsmittel, dadurch gekennzeichnet, dass es mindestens eine Verbindung gemäss einem der Ansprüche 1 bis 23 der Formel I, in freier Form oder gegebenenfalls in agrochemisch verwendbarer Salzform, als Wirkstoff und mindestens einen Hilfsstoff enthält.

29. Mittel gemäss Anspruch 28 zur Bekämpfung von Insekten und/oder Spinnentieren.

30. Verfahren zur Herstellung eines Mittels gemäss Anspruch 28 oder 29, dadurch gekennzeichnet, dass man den Wirkstoff mit dem (den) Hilfsstoff(en) innig vermischt.

31. Verfahren zur Bekämpfung von Schädlingen, dadurch gekennzeichnet, dass man als Wirkstoff eine Verbindung gemäss einem der Ansprüche 1 bis 23 der Formel I, in freier Form oder gegebenenfalls in agrochemisch verwendbarer Salzform, auf die Schädlinge oder ihren Lebensraum appliziert.

32. Verfahren gemäss Anspruch 31 zur Bekämpfung von Insekten und/oder Spinnentieren.

33. Verwendung einer Verbindung gemäss einem der Ansprüche 1 bis 23 der Formel I, in freier Form oder gegebenenfalls in agrochemisch verwendbarer Salzform, in einem Verfahren gemäss Anspruch 30 oder 31.

34. Verwendung einer Verbindung gemäss einem der Ansprüche 1 bis 23 der Formel I, in freier Form oder gegebenenfalls in agrochemisch verwendbarer Salzform, zur Herstellung eines Mittels gemäss Anspruch 28 oder 29.

35. Eine Verbindung der Formel 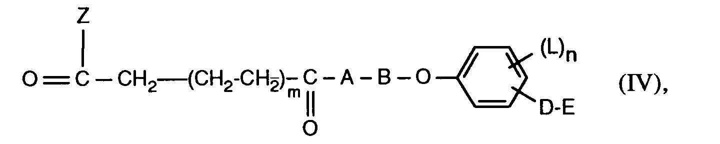 worin A, B, L, D, E, m und n die in Anspruch 1 für die Formel I angegebenen Bedeutungen haben und Z für Wasserstoff oder Methyl steht, in freier Form oder gegebenenfalls in Salzform.

## Claims

1. A compound of the formula 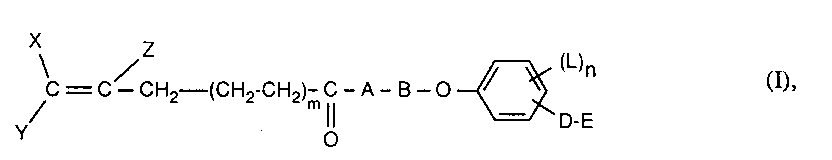 in which
A is oxygen, sulfur or -NR₁-;
B is C₂-C₆alkylene,
D-E is -O-E, -S-E, -O-CH₂-E, -O-C(=O)-E, -O-C(=O)-O-E, -O-C(=O)-N(H)-E or -O-C(=S)-N(H)-E;
E is phenyl; phenyl which is substituted by one to three substituents selected from the group consisting of halogen, C₁-C₄alkyl, halo-C₁-C₄alkyl, C₁-C₄alkoxy, halo-C₁-C₄alkoxy, cyano, nitro and methylenedioxy; a five-membered aromatic heterocycle which has one to three hetero atoms selected from the group consisting of nitrogen, oxygen and sulfur; a five-membered aromatic heterocycle which has one to three hetero atoms selected from the group consisting of nitrogen, oxygen and sulfur and which is substituted by one or two substituents selected from the group consisting of halogen, C₁-C₄alkyl and C₁-C₄haloalkyl; a six-membered aromatic heterocycle which has one to three nitrogen atoms; or a six-membered aromatic heterocycle which has one to three nitrogen atoms and which is substituted by one or two substituents selected from the group consisting of halogen, C₁-C₄alkyl and C₁-C₄haloalkyl;
L is halogen or methyl;
X is fluorine;
Y is chlorine or fluorine;
Z is hydrogen, fluorine or methyl;
m is the number zero, one, two, three, four or five;
n is the number zero, one or two and
R₁ is hydrogen, C₁-C₄alkyl, phenylthio or tolylthio, in free form or, where appropriate, in salt form.

2. A compound according to claim 1 of the formula I in which A is oxygen.

3. A compound according to claim 1 or 2 of the formula I in which B is an ethylene bridge.

4. A compound according to claim 1 of the formula I in which A is a bridge -NH-.

5. A compound according to any one of claims 1, 3 and 4 of the formula I in which n is zero.

6. A compound according to any one of claims 1 to 5 of the formula I in which D is -O-.

7. A compound according to any one of claims 1 to 6 of the formula I in which E is phenyl; pyridyl; thiadiazolyl; phenyl which is substituted by one or two substituents selected from the group consisting of cyano, nitro, halogen, C₁-C₄alkyl and C₁-C₄haloalkyl; pyridyl which is substituted by one or two substituents selected from the group consisting of halogen, C₁-C₄alkyl and halo-C₁-C₄alkyl, substituted pyridyl; or C₁-C₄alkylthiazolyl.

8. A compound according to any one of claims 1 to 7 of the formula I in which m is zero, one or four.

9. A compound according to any one of claims 1 to 8 of the formula I in which Y is fluorine and Z is hydrogen.

10. A compound according to any one of claims 1, 3 to 5 and 7 to 9 of the formula I in which D is -O-CH₂-.

11. A compound according to any one of claims 1 to 5 and 7 to 9 of the formula I in which D is -O-CO- or -O-CO-O-.

12. A compound according to claim 2 of the formula I in which n is zero.

13. A compound according to claim 12 of the formula I in which D is oxygen or sulfur.

14. A compound according to claim 2 of the formula I in which D is -O-CH₂-.

15. A compound according to claim 6 of the formula I in which E is a substituted or unsubstituted phenyl, thiadiazolyl or pyridyl group.

16. A compound according to claim 3 of the formula I in which A is oxygen, n is zero, D is oxygen, sulfur or -O-CH₂-, E is phenyl; pyridyl; thiadiazolyl; phenyl which is substituted by one or two substituents selected from the group consisting of cyano, nitro, halogen, C₁-C₄alkyl and C₁-C₄haloalkyl; pyridyl which is substituted by one or two substituents selected from the group consisting of halogen, C₁-C₄alkyl and halo-C₁-C₄alkyl; or C₁-C₄alkylthiadiazolyl, m is zero, one or four, Y is fluorine and Z is hydrogen.

17. A compound according to claim 16 of the formula I in which m is 1, B is -CH₂CH₂-, D-E is -O-CH₂-E and E is 4-trifluoromethylphenyl.

18. A compound according to claim 1 of the formula I selected from the group consisting of the compounds
2-(4-phenoxyphenoxy)ethyl 4,4-difluorobut-3-enoate,
2-(4-benzyloxyphenoxy)ethyl 4,4-difluorobut-3-enoate,
2-[4-(4-methyl)benzyloxyphenoxy]ethyl 4,4-difluorobut-3-enoate,
2-(4-phenoxyphenoxy)ethyl 6,6-difluorohex-5-enoate,
2-(4-benzyloxyphenoxy)ethyl 6,6-difluorohex-5-enoate,
2-(4-phenoxyphenoxy)ethyl 8,8-difluorooct-7-enoate,
2-(4-benzyloxyphenoxy)ethyl 8,8-difluorooct-7-enoate,
2-(4-phenoxyphenoxy)ethyl 10,10-difluorodec-9-enoate,
2-(4-benzyloxyphenoxy)ethyl 10,10-difluorodec-9-enoate,
2-(4-phenoxyphenoxy)ethyl 12,12-difluorododec-11-enoate and
2-(4-benzyloxyphenoxy)ethyl 12,12-difluorododec-11-enoate.

19. 2-[4-(4-Trifluoromethyl)benzyloxyphenoxy]ethyl 4,4-difluorobut-3-enoate according to claim 1.

20. 2-[4-(4-Trifluoromethyl)benzyloxyphenoxy]ethyl 6,6-difluorohex-5-enoate according to claim 1.

21. 2-[4-(4-tert-Butyl)benzyloxyphenoxy]ethyl 6,6-difluorohex-5-enoate according to claim 1.

22. 2-[4-(4-Trifluoromethyl)benzyloxyphenoxy]ethyl 12,12-difluorododec-11-enoate according to claim 1.

23. 2-[4-(4-tert-Butyl)benzyloxyphenoxy]ethyl 12,12-difluorododec-11-enoate according to claim 1.

24. A process for the preparation of a compound according to claim 1 of the formula I, which comprises
a) reacting a compound of the formula 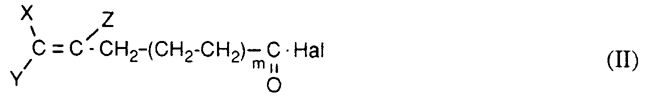 in which X, Y, Z and m are as defined in formula I and Hal is chlorine or bromine with a compound of the formula 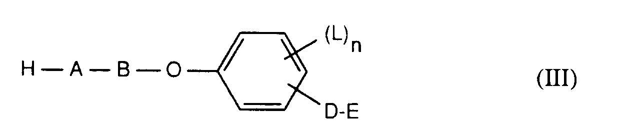 in which A, B, L, D, E and n are as defined in formula I, or, where appropriate, a salt thereof,
in the presence or absence of an inert solvent and in the presence of an acid-binding agent, or
b) reacting a compound of the formula 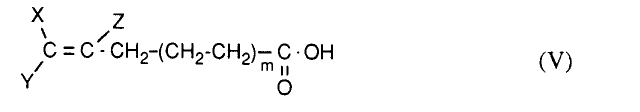 in which X, Y, Z and m are as defined in formula I
with a compound of the formula III or, where appropriate, a salt thereof,
in the presence or absence of an inert solvent and of a catalyst or a dehydrating agent, and in each case, if desired, converting a free compound of the formula I which can be obtained according to the process into a salt, or converting a salt of a compound of the formula I, which can be obtained according to the process, into the free compound of the formula I or into a different salt.

25. A process for the preparation of a compound according to claim 1 of the formula I in which A is oxygen or sulfur, which comprises reacting a compound of the formula 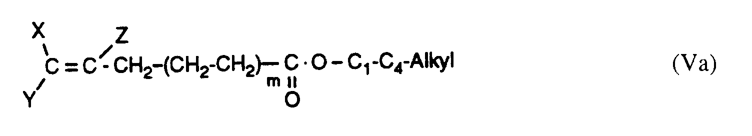 in which X, Y, Z and m are as defined in formula I
with a compound of the formula 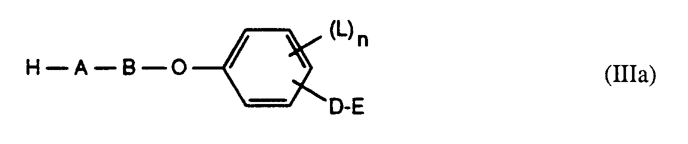 in which B, D, E, L and n are as defined in formula I and A is oxygen or sulfur with base or acid catalysis.

26. A process for the preparation of a compound according to claim 1 of the formula I in which A is NR₁, which comprises reacting a compound of the formula (Va) as defined in claim 25 with a compound of the formula 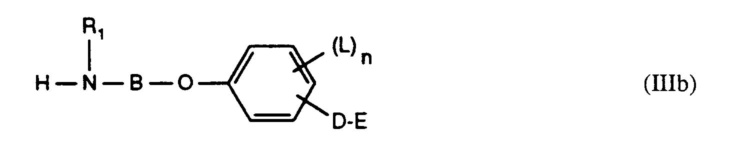 in which R₁, B, D, E, L and n are as defined in formula I, or a salt thereof.

27. A process for the preparation of a compound of the formula I in which Z is hydrogen or methyl, in free form of in salt form, which comprises reacting a compound of the formula 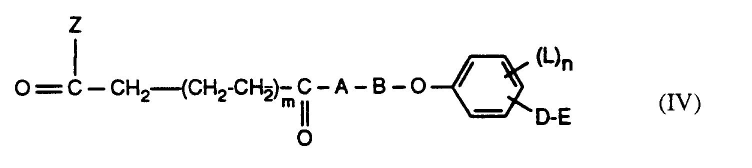 in which A, B, L, D, E, m and n are as defined in formula I and Z is hydrogen or methyl, or, where appropriate, a salt thereof,
with a halomethane or with an alkali metal trihaloacetate
in an inert solvent in the presence of a trisubstituted phosphane.

28. A pesticidal composition which comprises at least one compound according to any one of claims 1 to 23 of the formula I, in free form or, where appropriate, in agrochemically utilizable salt form, as active ingredient and at least one auxiliary.

29. A composition according to claim 28 for controlling insects and/or arachnids.

30. A process for the preparation of a composition according to claim 28 or 29, which comprises intimately mixing the active ingredient with the auxiliary, or auxiliaries.

31. A method of controlling pests, which comprises applying, as active ingredient, a compound according to any one of claims 1 to 23 of the formula I, in free form or, where appropriate, in agrochemically utilizable salt form, to the pests or their environment.

32. A method according to claim 31 for controlling insects and/or arachnids.

33. The use of a compound according to any one of claims 1 to 23 of the formula I, in free form or, where appropriate, in agrochemically utilizable salt form, in a process according to claim 30 or 31.

34. The use of a compound according to any one of claims 1 to 23 of the formula I, in free form or, where appropriate, in agrochemically utilisable salt form, for the preparation of a composition according to claim 28 or 29.

35. A compound of the formula 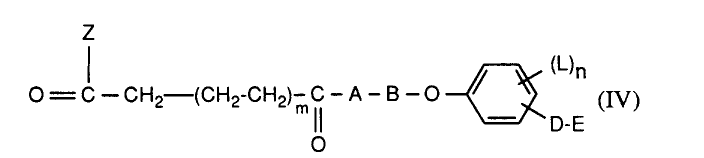 in which A, B, L, D, E, m and n are as defined in formula I in claim 1 and Z is hydrogen or methyl, in free form or, where appropriate, in salt form.

## Revendications

1. Un composé de formule 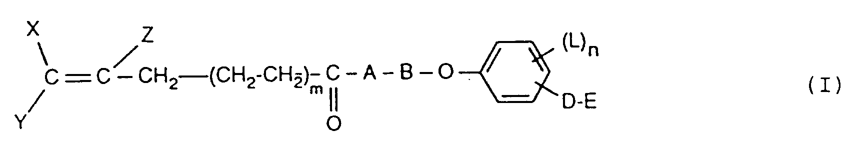 dans laquelle
A représente l'oxygène, le soufre ou -NR₁- ;
B représente un groupe alkylène en C2-C6,
D-E représente -O-E, -S-E, -O-CH₂-E, -O-C(=O)-E, -O-C(=O)-O-E, -O-C(=O)-N(H)-E ou -O-C(=S)-N(H)-E ;
E représente un groupe phényle ; un groupe phényle portant un à trois substituants choisis parmi les halogènes, les groupes alkyle en C1-C4, halogénoalkyle en C1-C4, alcoxy en C1-C4, halogénoalcoxy en C1-C4, cyano, nitro et méthylènedioxy ; un hétérocycle aromatique à cinq chaînons contenant un à trois hétéroatomes choisis parmi l'azote, l'oxygène et le soufre ; un hétérocycle aromatique à cinq chaînons contenant un à trois hétéroatomes choisis parmi l'azote, l'oxygène et le soufre et portant un ou deux substituants choisis parmi les halogènes, les groupes alkyle en C1-C4 et halogénoalkyle en C1-C4 ; un hétérocycle aromatique à six chaînons contenant un à trois atomes d'azote ; ou un hétérocycle aromatique à six chaînons contenant un à trois atomes d'azote et portant un ou deux substituants choisis parmi les halogènes, les groupes alkyle en C1-C4 et halogénoalkyle en C1-C4 ;
L représente un halogène ou un groupe méthyle ;
X représente le fluor ;
Y représente le fluor ou le fluor ;
Z représente l'hydrogène, le fluor ou un groupe méthyle ;
m est égal à 0, 1, 2, 3, 4 ou 5 ;
n est égal à 0, 1 ou 2 et
R₁ représente l'hydrogène, un groupe alkyle en C1-C4, phénylthio ou tolylthio, à l'état libre ou le cas échéant à l'état de sel.

2. Un composé selon revendication 1, répondant à la formule I dans laquelle A représente l'hydrogène.

3. Un composé selon revendication 1 ou 2, répondant à la formule I dans laquelle B représente un pont éthylène.

4. Un composé selon revendication 1, répondant à la formule I dans laquelle A représente un pont -NH-.

5. Un composé selon l'une des revendications 1, 3 ou 4, répondant à la formule I dans laquelle n est égal à 0.

6. Un composé selon l'une des revendications 1 à 5, répondant à la formule I dans laquelle D représente -O-,

7. Un composé selon l'une des revendications 1 à 6, répondant à la formule I dans laquelle E représente un groupe phényle ; un groupe pyridyle ; un groupe thiadiazolyle ; un groupe phényle portant un ou deux substituants choisis parmi les groupes cyano, nitro, les halogènes, les groupes alkyle en C1-C4 et halogénoalkyle en C1-C4 ; un groupe pyridyle portant un ou deux substituants choisis parmi les halogènes, les groupes alkyle en C1-C4 et halogénoalkyle en C1-C4 : ou un groupe (alkyle en C1-C4)thiazolyle.

8. Un composé selon l'une des revendications 1 à 7, répondant à la formule I dans laquelle m est égal à 0, 1 ou 4.

9. Un composé selon l'une des revendications 1 à 8, répondant à la formule I dans laquelle Y représente le fluor et Z l'hydrogène.

10. Un composé selon l'une des revendications 1, 3 à 5 et 7 à 9, répondant à la formule I dans laquelle D représente -O-CH₂-.

11. Un composé selon l'une des revendications 1 à 5 et 7 à 9, répondant à la formule I dans laquelle D représente -O-CO- ou -O-CO-O-.

12. Un composé selon revendication 12, répondant à la formule I dans laquelle n est égal à 0.

13. Un composé selon revendication 12, répondant à la formule I dans laquelle D représente l'oxygène ou le soufre.

14. Un composé selon revendication 2, répondant à la formule I dans laquelle D représente -O-CH₂-.

15. Un composé selon revendication 6, répondant à la formule I dans laquelle E représente un groupe phényle, thiadiazolyle ou pyridyle éventuellement substitué.

16. Un composé selon revendication 3, répondant à la formule I dans laquelle A représente l'oxygène, n est égal à 0, D représente l'oxygène, le soufre ou -O-CH₂-, E représente un groupe phényle ; pyridyle ; thiadiazolyle, un groupe phényle portant un ou deux substituants choisis parmi les groupes cyano, nitro, les halogènes, les groupes alkyle en C1-C4 et halogénoalkyle en C1-C4 ; un groupe pyridyle portant un ou deux substituants choisis parmi les halogènes, les groupes alkyle en C1-C4 et halogénoalkyle en C1-C4 ; ou un groupe (alkyle en C1-C4)thiadiazolyle, m est égal à 0, 1 ou 4, Y représente le fluor et Z l'hydrogène.

17. Un composé selon revendication 16, répondant à la formule I dans laquelle m est égal à 1, B représente -CH₂CH₂-, D-E représente -O-CH₂-E et E représente un groupe 4-trifluorométhylphényle.

18. Un composé selon revendication 1, répondant à la formule I, choisi parmi les suivants :
4,4-difluorobut-3-énoate de 2-(4-phénoxyphénoxy)éthyle,
4,4-difluorobut-3-énoate de 2-(4-benzyloxyphénoxy)éthyle,
4,4-difluorobut-3-énoate de 2-[4-(4-méthyl)benzyloxyphénoxy]éthyle,
6,6-difluorohex-5-énoate de 2-(4-phénoxyphénoxy)éthyle,
6,6-difluorohex-5-énoate de 2-(4-benzyloxyphénoxy)éthyle,
8,8-difluoroct-7-énoate de 2-(4-phénoxyphénoxy)éthyle,
8,8-difluoroct-7-énoate de 2-(4-benzyloxyphénoxy)éthyle,
10,10-difluorodéc-9-énoate de 2-(4-phénoxyphénoxy)éthyle,
10,10-difluorodéc-9-énoate de 2-(4-benzyloxyphénoxy)éthyle,
12,12-difluorodéc-11-énoate de 2-(4-phénoxyphénoxy)éthyle et
12,12-difluorodéc-11-énoate de 2-(4-benzyloxyphénoxy)éthyle.

19. Le 4,4-difluorobut-3-énoate de 2-[4-(4-trifluorométhyl)benzyloxyphénoxy]éthyle selon revendication 1.

20. Le 6,6-difluorohex-5-énoate de 2-[4-(4-trifluorométhyl)benzyloxyphénoxy]éthyle selon revendication 1.

21. Le 6,6-difluorohex-5-énoate de 2-[4-(4-tert-butyl)benzyloxyphénoxy]éthyle selon revendication 1.

22. Le 12,12-difluorododéc-11-énoate de Le 6,6-difluorohex-5-énoate de 2-[4-(4-trifluoro-méthyl)benzyloxyphénoxy]éthyle selon revendication 1.

23. Le 12,12-difluorododéc-11-énoate de Le 6,6-difluorohex-5-énoate de 2-[4-(4-tert-butyl)benzyloxy-phénoxy]éthyle selon revendication 1.

24. Procédé de préparation d'un composé selon revendication 1, répondant à la formule I, caractérisé en ce que
a) on fait réagir un composé de formule 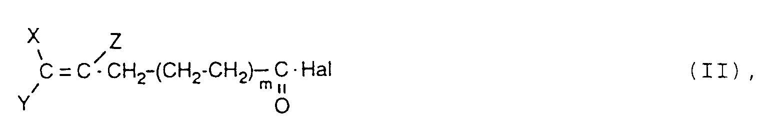 dans laquelle X, Y, Z et m ont les significations indiquées en référence à la formule I et Hal représente le chlore ou le brome, le cas échéant dans un solvant inerte et en présence d'un capteur d'acide, avec un composé de formule 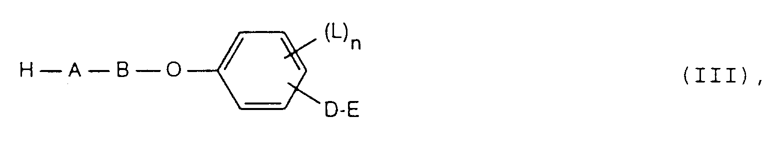 dans laquelle A, B, L, D, E et n ont les significations indiquées en référence à la formule I, ou bien, le cas échéant, l'un de ses sels, ou bien
b) on fait réagir un composé de formule 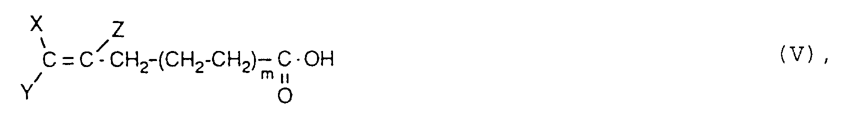 dans laquelle X, Y, Z et m ont les significations indiquées en référence à la formule I, le cas échéant en présence d'un solvant inerte et d'un catalyseur ou d'un agent déshydratant, avec un composé de formule III ou, le cas échéant, l'un de ses sels, et dans les deux cas, si on le désire, on convertit un composé de formule I obtenu à l'état libre en un sel ou un sel d'un composé de formule I en le composé libre de formule I ou en un autre sel.

25. Procédé de préparation d'un composé selon revendication 1, répondant à la formule I dans laquelle A représente l'oxygène ou le soufre, caractérisé en ce que l'on fait réagir un composé de formule 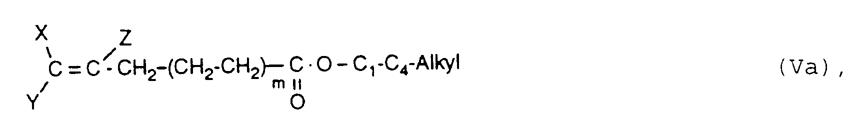 dans laquelle X, Y, Z et m ont les significations indiquées en référence à la formule I, avec catalyse par des bases ou des acides, avec un composé de formule 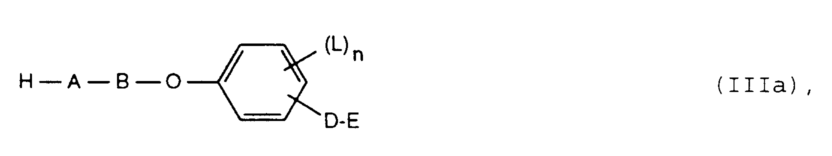 dans laquelle B, D, E, L et n ont les significations indiquées en référence à la formule I et A représente l'oxygène ou le soufre.

26. Procédé de préparation d'un composé selon revendication 1, répondant à la formule I dans laquelle A représente NR₁, caractérisé en ce que l'on fait réagir un composé de formule Va tel que défini dans la revendication 25, avec un composé de formule 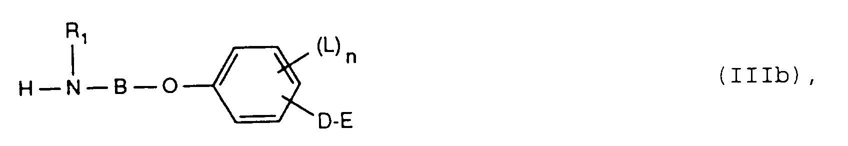 dans laquelle R₁, B, D, E, L et n ont les significations indiquées en référence à la formule I, ou l'un de ses sels.

27. Procédé de préparation d'un composé de formule I dans laquelle Z représente l'hydrogène ou un groupe méthyle, à l'état libre ou à l'état de sel, caractérisé en ce que l'on fait réagir un composé de formule 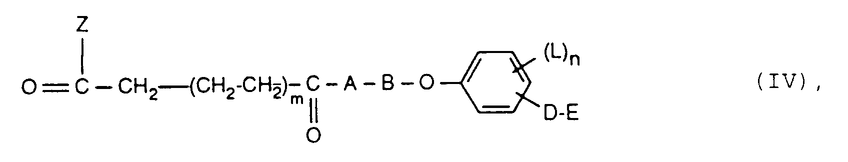 dans laquelle A, B, L, D, E, m et n ont les significations indiquées en référence à la formule I et Z représente l'hydrogène ou un groupe méthyle, ou le cas échéant l'un de ses sels, dans un solvant inerte, en présence d'un phosphate trisubstitué, avec un halogénométhane ou un sel alcalin d'un acide trihalogénoacétique.

28. Produit parasiticide, caractérisé en ce qu'il contient en tant que substance active au moins un composé selon l'une des revendications 1 à 23 répondant à la formule I, à l'état libre ou le cas échéant à l'état de sel convenant pour les applications agrochimiques, et au moins un produit auxiliaire.

29. Produit selon revendication 28, pour la lutte contre les insectes et/ou les arachnides.

30. Procédé de préparation d'un produit selon revendication 28 ou 29, caractérisé en ce que l'on mélange intimement la substance active avec le ou les produits auxiliaires.

31. Procédé pour combattre les parasites, caractérisé en ce que l'on applique sur les parasites ou leur habitat, en tant que substance active, un composé selon l'une des revendications 1 à 23, répondant à la formule I, à l'état libre ou le cas échéant à l'état de sel convenant pour les applications agrochimiques.

32. Procédé selon revendication 31, pour la lutte contre les insectes et/ou les arachnides.

33. Utilisation d'un composé selon l'une des revendications 1 à 23, répondant à la formule I, à l'état libre ou le cas échéant à l'état de sel convenant pour les applications agrochimiques, dans un procédé selon revendication 30 ou 31.

34. Utilisation d'un composé selon l'une des revendications 1 à 23, répondant à la formule I, à l'état libre ou le cas échéant à l'état de sel convenant pour les applications agrochimiques, pour la Préparation d'un produit selon revendication 28 ou 29.

35. Un composé de formule 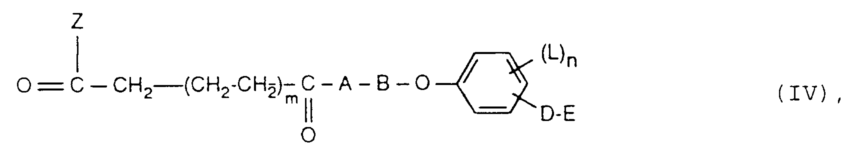 dans laquelle A, B, L, D, E, m et n ont les significations indiquées dans la revendication 1 en référence à la formule I et Z représente l'hydrogène ou un groupe méthyle, à l'état libre ou le cas échéant à l'état de sel.
